# FASCICULE DE BREVET EUROPEEN

(11) **EP 2 429 658 B1**
(45) Date de publication et mention de la délivrance du brevet: **20.04.2016**
(21) Numéro de dépôt: 10723642.4
(22) Date de dépôt: 12.05.2010
(51) Int. Cl.: A61P 31/04, A61K 39/095, A61K 9/127

(54) **Procédé pour adjuver le lipopolysaccharide (LPS) des bactéries à gram-négatif**
Verfahren zur Adjuvierung des Lipopolysaccharids (LPS) von gram-negativen Bakterien
Method for adjuvating the lipopolysaccharide (LPS) of gram-negative bacteria

(30) Priorité: 14.05.2009 FR 0902330; 29.07.2009 US 229577 P
(43) Date de publication de la demande: 21.03.2012
(73) Titulaire: Sanofi Pasteur, 69367 Lyon Cedex 07 (FR)
(72) Inventeur: HAENSLER, Jean, F-69290 Grezieu la Varenne (FR); GUY, Bruno, F-69002 Lyon (FR)
(86) Numéro de dépôt international: PCT/FR2010/000365
(87) Numéro de publication internationale: WO 2010/130896

(56) Documents cités:
- WO-A-00/25811
- WO-A-2006/108586
- US-A1- 2003 059 444
- TAHA ET AL: "Neisseria meningitidis : actualites biologiques", REVUE FRANCOPHONE DES LABORATOIRES, ELSEVIER, AMSTERDAM, NL, vol. 2009, no. 409, 1 février 2009 (2009-02-01), pages 36-38, XP025967441, ISSN: 1773-035X [extrait le 2009-02-01]
- HOLST J ET AL: "The concept of ''tailor-made'', protein-based, outer membrane vesicle vaccines against meningococcal disease", VACCINE, BUTTERWORTH SCIENTIFIC. GUILDFORD, GB, vol. 23, no. 17-18, 18 mars 2005 (2005-03-18), pages 2202-2205, XP004777523, ISSN: 0264-410X
- SPOHN R ET AL: "Synthetic lipopeptide adjuvants and Toll-like receptor 2-structure-activity relationships", VACCINE, BUTTERWORTH SCIENTIFIC. GUILDFORD, GB, vol. 22, no. 19, 23 juin 2004 (2004-06-23) , pages 2494-2499, XP004515469, ISSN: 0264-410X
- PETROV A B ET AL: "TOXICITY AND IMMUNOGENICITY OF NEISSERIA-MENINGITIDIS LIPOPOLYSACCHARIDE INCORPORATED INTO LIPOSOMES", INFECTION AND IMMUNITY, vol. 60, no. 9, 1992, pages 3897-3903, XP002558878, ISSN: 0019-9567

## Description

L'invention s'inscrit dans le domaine des vaccins et a pour objet un procédé permettant d'accroire la réponse immune d'un mammifère à l'encontre d'un antigène vaccinal d'intérêt : le lipopolysaccharide (LPS) des bactéries à Gram-négatif.

Le LPS est un constituant majeur de la membrane externe de la paroi des bactéries à Gram-négatif. Le LPS est toxique à forte dose pour les mammifères et au regard de cette activité biologique, a été dénommé endotoxine. If est responsable du choc septique, pathologie mortelle qui se développe suite à une infection aiguë par une bactérie à Gram-négatif.

La structure du LPS est constituée d'une partie lipidique, appelée le lipide A, liée de manière covalente à une partie polysaccharidique.

Le lipide A est responsable de la toxicité du LPS. Il est fortement hydrophobe et permet d'ancrer le LPS dans la membrane externe de la paroi. Le lipide A est composé d'une structure disaccharidique substituée par des chaînes d'acides gras. Le nombre et la composition des chaînes d'acides gras varient d'une espèce à l'autre.

La partie polysaccharidique est constituée par des chaînes de carbohydrates qui sont responsables de l'antigénicité. On distingue au moins 3 grandes régions dans cette partie polysaccharidique :
(i) un core interne, constitué de monosaccharides [un ou plusieurs KDO (acide 2-céto-3-desoxy-octulosonique) et un ou plusieurs heptose (Hep)] invariants au sein d'une même espèce bactérienne ;
(ii) un core externe lié à un heptose et constitué de divers monosaccharides ; et
(iii) une chaîne externe O-spécifique constituée d'un enchaînement d'unités répétitives - unités répétitives elles-mêmes composées de un ou plusieurs monosaccharides différents.

La composition de la partie polysaccharidique varie d'une espèce à une autre, d'un sérotype (immunotype chez le méningocoque) à un autre au sein d'une même espèce.

Chez un certain nombre de bactéries non-entériques à Gram-négatif telles que les *Neisseriae, Bordetellae, Branhamellas, Haemophilus* et *Moraxellae,* la chaîne O-spécifique n'existe pas. La partie saccharidique de LPS de ces bactéries n'est constituée que du core oligosaccharidique). Par conséquent, le LPS de ces bactéries est souvent dénommé lipooligosaccharide (LOS).

Le LPS n'est pas seulement toxique, il est aussi immunogène. Chez les mammifères, les anticorps anti-LPS sont générés pendant le portage et l'infection et peuvent être protecteurs. Ainsi, l'usage du LPS a déjà été envisagé dans la prophylaxie des infections dues aux bactéries à Gram-négatif et des maladies associées.

Le LPS n'est pas le seul constituant des bactéries à Gram-négatif à avoir été proposé comme antigène vaccinal. Certaines protéines de ces bactéries l'ont été de même, et notamment certaines protéines de leur membrane externe.

Un moyen commode pour obtenir une composition vaccinale consiste à préparer des OMVs (outer membrane vesicles ou vésicules de membrane externe) en traitant avec un détergent (Octyl-glucoside) des blebs d'une préparation bactérienne. Les constituants des OMVs sont multiples : entre autre du LPS résiduel et les protéines de la membrane externe. De nombreuses publications (par exemple, TAHA M-K (2008) revue francophone des laboratoires, supplément au numéro 399, pp. 36-38; US 2003 059444; WO 00 25811) témoignent de l'intérêt de cette approche.

Néanmoins, l'approche OMV comporte aussi quelques désavantages. Ainsi, face aux exigences accrues de la réglementation, la caractérisation et la quantification des différents composants des OMVs et plus encore, la possibilité de fabriquer les lots à l'identique restent un problème délicat.

Afin de maîtriser plus aisément le contenu d'un vaccin, d'autres approches ont été élaborées.

Une de ces approches alternatives consiste à purifier du LPS et à le détoxifier. Différentes méthodes de détoxification sont déjà connues : chimique, génétique, enzymatique ou bien encore par complexation avec un peptide ou inclusion en liposomes. Une fois détoxifié, le LPS peut être conjugué à un polypeptide ou peptide porteur.

En vue de fabriquer un vaccin, le LPS purifié et détoxifié peut être utilisé seul ou en mélange, notamment avec une ou des protéines d'intérêt vaccinal et plus particulièrement avec une ou des protéines de membrane externe. Les protéines auront été au préalable obtenues par voie recombinante ou sous forme native puis purifiées.

Bien que le LPS soit réputé immunogène, l'adjonction d'un adjuvant - c'est-à-dire d'une molécule capable d'accroire la réponse immune d'un organisme à l'égard d'une autre molécule (ici, le LPS) - peut s'avérer très bénéfique. Reste à sélectionner parmi tous les adjuvants connus, celui qui fonctionnera avec le LPS de manière optimale.

Le document par Petrov A et al (1992), Infection and Immunity, vol.60 pp.3897-3903 décrit une diminution de la toxicité du lipopolysaccharide (LPS) par incorporation dans des liposomes, et l'effet adjuvant des liposomes sur la réponse immunitaire dirigée contre le LPS.

Dans le domaine des vaccins, un des enjeux majeurs des prochaines années sera notamment la mise sur le marché d'un vaccin destiné à prévenir l'ensemble des infections à *Neisseria meningitidis. N. meningitidis* est responsable d'un certain nombre de pathologies, parmi lesquelles de façon dominante, les méningites et les méningococcies, mais aussi des arthrites et péricardites. Les méningococcies peuvent se compliquer de *purpura fulminans* et de choc septique mortel.

D'une manière générale, les méningites sont soit d'origine virale, soit d'origine bactérienne. Dans les pays développés, les bactéries principalement responsables sont : *N. meningitidis* et *Streptococcus pneumoniae,* respectivement impliquées dans environ 40 et 50 % des cas de méningites bactériennes. Dans les pays en voie de développement *Haemophilus influenzae* reste aussi une source importante de méningites.

On dénombre en France, environ 600 à 800 cas par an de méningites à *N. meningitidis.* Aux Etats-Unis, le nombre de cas s'élève à environ 2 500 à 3 000 par an.

L'espèce *N. meningitidis* est subdivisée en sérogroupes selon la nature des polysaccharides de capsules. Bien qu'il existe une douzaine de sérogroupes, 90 % des cas de méningites sont attribuables aux sérogroupes : A, B, C, Y et W135.

Il existe des vaccins efficaces à base de polysaccharides capsulaires pour prévenir les méningites à *N. meningitidis* sérogroupes A, C, Y et W135. Ces polysaccharides tels quels ne sont que peu ou pas immunogènes chez les enfants de moins de 2 ans et n'induisent pas de mémoire immunitaire. Toutefois, ces inconvénients peuvent être surmontés en conjuguant ces polysaccharides à une protéine porteuse.

Par contre, le polysaccharide de *N. meningitidis* groupe B n'est pas ou peu immunogène chez l'homme, qu'il soit sous forme conjuguée ou non (Bruge et al, Vaccine (2004) 22 : 1087). Ainsi, il apparaît hautement souhaitable de rechercher un vaccin à l'encontre des méningites induites par *N. meningitidis* notamment du sérogroupe B autre qu'un vaccin à base de polysaccharide capsulaire.

A cette fin, différentes protéines de la membrane externe de *N. meningitidis* ont déjà été proposées comme antigène vaccinal ainsi que le LPS. Un nombre assez important de protéines de *N. meningitidis* ont déjà fait l'objet de travaux d'investigation. On cite notamment le récepteur de la transferrine humaine qui est composé de deux sous-unités TbpA et TbpB.

On a maintenant mis en évidence dans des modèles souris et lapin, que la réponse immune à l'encontre du lipopolysaccharide (LPS) pouvait être améliorée lorsque le LPS est administré avec la forme lipidée de la sous-unité B dé la protéine liant la transferrine humaine (TbpB) de *N. meningitidis,* souche M982, produite par voie recombinante. Ceci a été observé en employant diverses formes de LPS. Pour observer cet effet, il suffit que le LPS et la TbpB soient mis en présence l'un de l'autre, sous quelle que forme que ce soit, *i.a.* simple mélange, protéoliposomes ou bien encore conjugué. Pour tenter d'expliquer les résultats, on postule que l'effet adjuvant de la TbpB sur le LPS est, au moins en partie, dû à l'association dans une même molécule, d'une chaîne d'acides gras jouant notamment le rôle d'agoniste du récepteur Toll-like 2 (TLR2) et d'épitopes T-helper.

En d'autres termes, les résultats générés, tels que reportés dans la partie expérimentale de la présente demande de brevet, nous enseignent que la lipoprotéine TbpB est capable d'adjuver le LPS.

C'est pourquoi, l'invention a pour objet un procédé pour adjuver du LPS d'une bactérie à gram-négatif selon lequel :
(i) on met en mélange du LPS ou des liposomes LPS (LPS formulé en liposomes) avec la sous-unité B lipidée du récepteur de la transferrine humaine (TbpB lipidée) de *Neisseria meningitidis* ou un fragment lipidé de la-dite sous-unite B; ou
(ii) on formule ensemble en liposomes du LPS et la TbpB lipidée de *N. meningitidis* ou un fragment lipidé de la-dite TbpB; ou
(iii) on conjugue du LPS avec la TbpB lipidée de *N. meningitidis* ou un fragment lipidé de la-dite TbpB;
et dans lequel chacunes des alternatives (i), (ii) et (iii) permet d'obtenir une préparation qui ne contient pas d'OMVs et qui est capable d'induire après administration à un mammifère, une réponse immune anti-LPS qui est améliorée par comparaison à la réponse immune anti-LPS observée après administration de la préparation correspondante dans laquelle la TbpB lipidée de *N. meningitidis* ou un fragment lipidé de cette dernière est omis.

On comprend aisément que la préparation ainsi obtenue ne contient pas non plus de protéosomes (mélange de protéines membranaires indéfini) directement préparés à partir de leur origine bactérienne naturelle. Ainsi, les porines telles que PorA et PorB, qui sont des protéines majeures de la membrane externe des bactéries à Gram-négatif, sont absentes, *i*.*a*. sous leur forme non-recombinante, de préférence définitivement absentes sous quelque forme que ce soit, d'une préparation obtenue par le procédé selon l'invention.

En d'autres termes, l'invention a pour objet l'utilisation de la TbpB lipidée de *N. meningitidis* ou un fragment lipidé de cette dernière, à titre d'adjuvant d'un LPS d'une bactérie à Gram-négatif - c'est-à-dire, pour améliorer la réponse immune d'un mammifère à l'encontre d'un LPS d'une bactérie à Gram-négatif par comparaison avec la réponse immune anti-LPS observée en l'absence de TbpB lipidée ou d'un fragment lipidé de cette dernière.

Dans la suite du texte, le terme « ou un fragment lipidé de cette dernière » n'apparaît plus, par souci de simplification. Néanmoins il reste sous-entendu chaque fois que le terme « TbpB lipidée » ou « TbpB » apparaît.

L'objectif auquel tend la présente invention - c'est-à-dire l'adjuvantation du LPS - se matérialise par une réponse immune anti-LPS améliorée. Cette amélioration peut se constater dans un ou plusieurs champ(s) de la réponse immune spécifique, notamment dans le champ de la réponse humorale aussi appelée réponse anticorps et/ou dans le champ de la réponse cellulaire.

Il peut s'agir d'une production accrue d'un ou plusieurs type(s) ou de sous-type(s) d'anticorps, IgA, IgM et IgG incluant les sous-classes d'IgG capables de fixer le complément.

Il peut s'agir aussi du renforcement d'une propriété recherchée, par exemple, du caractère bactéricide des anticorps qui peut être commodément apprécié à l'encontre de la souche d'origine du LPS (souche homologue). Sous un aspect avantageux, le caractère bactéricide des anticorps s'exercera aussi à l'encontre d'au moins une souche hétérologue.

De manière typique, l'analyse de la réponse anticorps ainsi que l'évaluation du caractère bactéricide peut se faire à la suite d'une ou plusieurs administration(s) de la préparation obtenue par le procédé selon l'invention ; de manière avantageuse après deux ou trois administrations consécutives espacées dans le temps. Selon un mode avantageux, on prélève un échantillon de sang peu de temps avant la première administration (par exemple, 1 à 7 jours avant) ; puis on procède à une ou des administrations à 2 à 8 semaines d'intervalle selon le mammifère receveur. Un échantillon de sang est prélevé 2 à 4 semaines après la dernière administration.

En présence de la TbpB de *N. meningitidis* ou d'un fragment lipidé de cette dernière , la réponse anticorps anti-LPS de type IgA, IgM ou IgG, telle qu'elle peut être mesurée, par exemple en unité ELISA, est accrue après la dernière administration, e.g. la deuxième ou troisième administration, au moins d'un facteur 1.2, 1.4, 1.6; de préférence d'au moins un facteur 1.8, 2, 2.2, 2.4, 2.6, 2.8, 3 ou plus.

Il existe plusieurs méthodes connues pour apprécier et exprimer la bactéricidie ; l'une d'entre elles est décrite dans la partie expérimentale.

D'une manière générale, on administre à des individus, par exemple des animaux, une préparation à tester, une ou plusieurs fois avec un certain délai d'intervalle. Les sera des individus sont prélevés ; puis leur activité bactéricide (bactéricidie) mesurée.

Selon la méthode décrite dans la partie expérimentale, on exprime le titre bactéricide d'un sérum comme étant l'inverse de la dilution du sérum donnant 50 % de mort bactérienne par comparaison avec le témoin « complément ». Puis on calcule le titre moyen global (GMT) des sera prélevés à un temps identique à partir d'un même groupe d'individus - c'est-à-dire d'individus ayant tous reçu le même produit. Enfin, pour chaque individu, on établit le taux de séroconversion comme étant *e.g*., le rapport titre bactéricide au temps T après administration : titre bactéricide avant administration. Pour chaque groupe d'individus, le taux de séroconversion qui le caractérise est établi comme étant le rapport GMT au temps T après administration: GMT avant administration. Ainsi le taux de séroconversion mesure l'accroissement du titre bactéricide. De manière alternative, le taux de séroconversion peut être aussi exprimé en « *fold increase* » comme étant le rapport GMT des sérums des animaux immunisés avec l'antigène : GMT des sérums des animaux recevant le contrôle négatif.

La bactéricidie est testée à l'encontre d'une souche de même immunotype que le LPS de la préparation à tester. Cette bactéricidie est jugée satisfaisante si après la dernière administration :
(i) le pourcentage d'individus ayant reçu la préparation à tester et présentant un accroissement du titre bactéricide d'au moins un facteur 3, de préférence 4 (comparé à celui mesuré avant toute administration) est supérieur à 60 %, de manière avantageuse à 70 %, de préférence à 80 % du total d'individus testés, de manière particulièrement préférée égal à 100 % du total d'individus testés ; ou, de préférence et,
(ii) le taux de séroconversion caractérisant un groupe d'individus ayant reçu la préparation à tester est supérieur à 3 ou 4, de manière avantageuse à 10, de préférence à 50, de manière particulièrement préférée à 75.

### Le LPS

Le LPS pouvant être adjuvanté par le procédé selon l'invention peut être n'importe quel LPS de bactérie à Gram-négatif, qu'elles soient entériques ou non-entériques, de préférence pathogènes. Sous un aspect particulier, il peut s'agir du LPS de bactéries non-entériques des genres tels que les *Neisseriae*, *Bordetellae, Branhamellas, Haemophilus* et *Moraxellae.* Le LPS de ces bactéries est aussi désigné sous le terme de LOS (lipooligosaccharide) en raison de l'absence de polysaccharide O-spécifique. A titre d'exemple additionnel, on cite le LPS/LOS des genres ou espèces *Klebsiella, Pseudomonas, Burkolderia, Porphyromonas, Franciscella, Yersinia, Enterobacter, Salmonella, Shigella, E. coli ;* et tout particulièrement le LOS de *N. meningitidis.*

Les souches de *N. meningitidis* sont classées en plusieurs immunotypes (IT L1 à L13), en fonction de leur réactivité avec une série d'anticorps qui reconnaissent des épitopes variés sur le LOS (Achtman et al, 1992, J. Infect. Dis. 165 : 53-68). Par la suite, le LOS de ces souches est répertorié de même en 13 immunotypes (IT L1 à L13). Les différences entre immunotypes proviennent de variations dans la composition et dans la conformation des chaînes oligosaccharidiques. Ceci transparaît dans le tableau ci-dessous, dérivé du tableau 2 de Braun et al, Vaccine (2004) 22 : 898, complété par des données obtenues ultérieurement et relatives aux immunotypes L9 (Schoudhury et al, Carbohydr. Res. (2008) 343 : 2771) et L11 (Mistretta et al, (2008) Poster at the 16th International Pathogenic Neisseria Conférence, Rotterdam) :

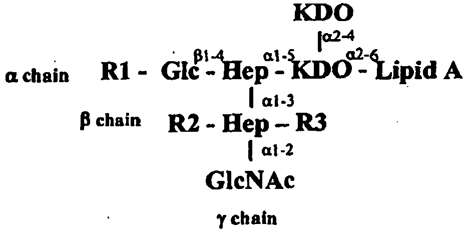

| **IT** | **chaîne α (incluant le substituent R1)** | **R1** | **R2** |
|---|---|---|---|
| **L1** | NeuNAcα2-6Galα1-4Galβ1-4Glcβ1-4 | PEA-3 | - |
| **L2** | NeuNAcα2-3Galβ1-4GlcNAcβ1-3Galβ1-4-Glcβ1-4 | Glcα(1-3)** | PEA-6 ou PEA-7 |
| **L3** | NeuNAcα2-3Galβ1-4GlcNAcβ1-3Galβ1-4-Glcβ1-4 | PEA-3 | - |
| **L4** | NeuNAcα2-3Galβ1-4GlcNAcβ1-3Galβ1-4 Glcβ1-4 | - | PEA-6 |
| **L5** | NeuNAcα2-3Galβ1-4GlcNAcβ1-3Galβ1-4Glcβ1-4Glcβ1-4 | Glca (1-3) | - |
| **L6** | GlcNAcβ1-3Galβ1-4 Glcβ1-4 | - | PEA-6 ou PEA-7 |
| **L7** | Galβ1-4GlcNAcβ1-3Galβ1-4 Glcβ1-4 Glcβ1-4 | PEA-3 | - |
| **L8** | Galβ1-4 Glcβ1-4 | PEA-3 | - |
| **L9** | Galβ1-4GlcNAcβ1-3Galβ1-4 Glcβ1-4 | - | PEA-6 |
| **L10** | n.d. | n.d. | n.d. |
| **L11** | Glcβ1-4Glcβ1-4 | PEA-3 | PEA-6. |
| **L12** | n.d | n.d. | n.d. |
| **L13** | n.d | n.d. | n.d. |

| | | | |
|---|---|---|---|
| n.d. : non déterminé. ** : Lorsque R2 est un résidu glucose, R2 est communément appelé chaîne β. | | | |

Entre autre, on peut noter que certains LOS peuvent être sialylés (présence d'acide N-acétyl neuraminique sur le résidu galactose (Gal) terminal de la chaîne α). Ainsi, les immunotypes L3 et L7 ne diffèrent que par la présence / absence respective de cette sialylation. Par ailleurs, la plupart des LOS sont substitués par un groupement O-acétyle sur le résidu glucosamine (α-GlcNAc ou chaîne γ) du core interne (Wakarchuk et al. (1998) Eur. J.. Biochem. 254 : 626 ; Gamian et al. (1992) J. Biol. Chem. 267 : 922 ; Kogan et al (1997) Carbohydr. Res. 298 : 191 ; Di Fabio et al. (1990) Can. J. Chem. 68 : 1029 ; Michon et al. (1990) J. Biol. Chem. 275 : 9716 ; Choudhury et al. (supra) ; et Mistretta et al. (supra).

Le motif de carbohydrates Galβ1-4GlcNAcβ1-3Galcβ1-4Glcβ1-4 ou motif lacto-N-neotetraose qui est présent dans la chaîne α de certains immunotypes du LPS de *N*. *meningitidis,* constitue un épitope qui peut potentiellement réagir de manière croisée avec les érythrocytes humains. Ainsi, en vue de fabriquer un vaccin destiné aux humains, il convient de choisir un LPS ne possédant pas ce motif. Il peut donc être particulièrement avantageux d'utiliser un LOS d'une souche de *N. meningitidis* d'immunotype L8 (appelé par la suite, LOS d'immunotype L8), notamment de la souche A1 de *N. meningitidis* (Zhu, Klutch & Tsai, FEMS Microbiology Letters (2001) 203 : 173 ainsi que Gu, Tsai & Karpas, J. Clin. Microbiol. (Aug 1992) 30 (8) : 2047) qui est publiquement disponible au sein de la communauté scientifique.

De manière alternative, on peut aussi envisager de partir par exemple d'une souche d'immunotype L2 ou L3 dans laquelle on a inactivé par mutation un gène intervenant dans la biosynthèse de la chaîne α, de manière à obtenir une structure LNnT incomplète. De telles mutations sont proposées dans la demande de brevet WO 04/014417. Il s'agit d'éteindre par mutation les gènes *lgt*B, *lgt*E (ou *Igt*H), *lgtA* ou *lgtA* et *Igt*C. Ainsi, il apparaît possible et intéressant d'utiliser un LPS provenant d'une souche de *N. meningitidis* d'immunotype L2 ou L3 *Igt*B⁻, *lgtE⁻* (ou *lgt*H⁻), *Igt*A⁻ ou *lgtA*⁻ et *IgtC*⁻.

Aux fins de la présente invention, le LPS peut être obtenu par des moyens conventionnels : en particulier, il peut être extrait à partir d'une culture bactérienne ; puis purifié selon des méthodes classiques. De nombreux procédés d'obtention sont décrits dans la littérature. A titre d'exemple, on cite *i*.*a*., Westphal & Jann, (1965) Meth. Carbohydr. Chem. 5 : 83 ; Gu & Tsaï, 1993, Infect. Immun. 61 (5) : 1873 ; Wu et al, 1987, Anal. Biochem. 160 : 281 and US 6,531,131. Une préparation de LPS peut être quantifiée selon des procédures bien connues. Le dosage du KDO par chromatographie d'échange d'anions à haute performance (HPAEC-PAD) est une méthode qui convient tout particulièrement.

### La TbpB de N. meningitidis

La TbpB de *N. meningitidis* telle que naturellement produite par N. *meningitidis,* est une lipoprotéine. Néanmoins, elle peut être avantageusement produite par voie recombinante dans un système d'expression qui permet notamment d'assurer la lipidation du polypeptide au sein même de l'organisme chargé de l'expression. Selon un mode préféré, la TbpB lipidée est une TbpB recombinante - c'est-à-dire produit par voie recombinante, *e*.*g*. dans un système d'expression hétérologue.

Un système d'expression met typiquement en oeuvre une cassette d'expression et une cellule hôte procaryote ou eucaryote (levure). La cassette d'expression code pour un précurseur de la TbpB (aussi appelé pro-TbpB). Ce précurseur est constitué d'une séquence signal caractéristique d'une lipoprotéine et de la séquence de la protéine mature ayant un résidu Cystéine en position N-terminale. Les trois acides aminés en position C-terminale de la séquence signal ainsi que le résidu Cystéine en position N-terminale de la séquence mature constitue le site de clivage (aussi appelé lipobox) : Cette lipobox a typiquement pour séquence : Leu - Ser/Ala - Ala/Gly - Cys. Une séquence signal typique est celle de la lipoprotéine Lpp d'*E*. *coli* : Met - Lys - Ala - Thr - Lys - Leu - Val - Leu - Glu - Ala - Val - De - Leu - Glu - Ser - Thr - Leu - Leu - Ala - Gly. Ainsi, dans la cassette d'expression, la séquence de polynucléotide codant pour la séquence d'acides aminés de la TbpB est fusionnée en 5' à une séquence signal appropriée.

Selon un mode particulièrement préféré, le LPS et la TbpB lipidée proviennent tous deux de la même espèce bactérienne, soit *N. meningitidis*.

La TbpB de *N. meningitidis* est comme toute protéine, définie par une séquence d'acides aminés. A l'intérieur de l'espèce, cette séquence d'acides aminés peut présenter un certain degré de variabilité sans que cela porte atteinte à la fonction biologique de la lipoprotéine. On parle alors de « variant allélique ». A une TbpB de *N*. *meningitidis,* correspondent une multiplicité de séquences présentant entre elles un certain degré d'identité, chacune des séquences provenant d'une souche particulière, l'une étant le variant allélique de l'autre.

Ainsi, on comprendra aisément que la présente invention ne se limite pas à l'emploi d'une TbpB particulière, définie par une séquence d'acides aminés particulière. Toute référence à une séquence d'acides aminés est faite à titre illustratif, non-limitatif.

La présente invention ne se limite pas non plus à une TbpB lipidée de forme sauvage. En effet, il peut s'agir non seulement d'une forme sauvage, mais aussi d'une forme mutée par addition, substitution ou délétion d'un ou plusieurs acides aminés.

Pour usage dans la présente invention, un fragment lipidé de la TbpB *de N. meningitidis,* est avantageusement le fragment N-terminal lipidé de la TbpB. La partie « polypeptide » du fragment peut avantageusement comporter un ou des épitopes T-helper - c'est-à-dire des épitopes capables d'être reconnus par les cellules-T helper - et de les activer. Avantageusement, il s'agit d'épitopes T-helper caractéristiques de l'organisme auquel le vaccin à base de LPS est destiné (un mammifère, notamment un humain) - c'èst-à-dire d'épitopes capables d'être reconnus par les cellules-T helper de l'organisme receveur et de les activer.

Le cadre de lecture ouvert (ORF) codant pour la TbpB (*tbp*B) de plusieurs souches de *N. meningitidis* a déjà été identifié par sa séquence ; et la séquence d'acides aminés de la protéine correspondante déduite. Ainsi les séquences *tbp*B et TbpB des souches de *N. meningitidis* de serogroupe B, M982 et B16B6, sont divulguées dans la demande de brevet EP 586 266. Celles des souches de méningoccoque MC58 (sérogroupe B), Z2491 (sérogroupe A) et FAM18 (sérogroupe C) sont respectivement divulguées dans Tettelin et al, Science, March 2000, 287 : 1809 ou WO 00/66791; Parkhill et al, Nature (March 2000) 404 : 502 ; et Bentley et al, PLoS Genet., 3, e23 (2007).

L'identification de ces dernières séquences ayant été faite dans le cadre du séquençage complet des génomes, un numéro d'ordre leur a été attribué. Ainsi, dans Tettelin et al *(supra)* ou WO 00/66791, les séquences *tpbB* / TbpB de la souche MC58 sont désignées sous la référence NMB 0460. Dans la suite du texte, on pourra désigner les protéines de *N. meningitidis* sans que cela fasse référence de manière limitative aux séquences de la souche MC58.

Chez *N. meningitidis,* on a documenté à ce jour deux grandes familles de TbpB : l'isotype I caractérisé par un gène *tbp*B de 1.8 kb et l'isotype II caractérisé par un gène *tbp*B de 2.1 kb. L'isotype I s'exprime dans le complexe clonal ST-11 et le II dans les complexes clonaux ST-8, ST-18, ST-32, ST-41/44 (Harrison et al, BMC Microbiol. 2008, 8 : 66). Les souches B16B6 (sérogroupe B) et FAM18 (sérogroupe C) sont des représentants de l'isotype I ; les souches M982, BZ83 et 8680 sont des représentants de l'isotype II. Aux fins de l'invention, les TbpBs de chacun des deux isotypes peuvent être utilisées de manière indifférente.

Sous un premier aspect du procédé selon l'invention, on mélange du LPS avec la TbpB. Le LPS peut être (i) simplement purifié, (ii) sous forme de conjugué - c'est-à-dire lié de manière covalente à un polypeptide porteur, ou bien encore (iii) formulé en liposomes.

Le LPS peut être obtenu sous forme purifiée par des moyens conventionnels ; en particulier, il peut être extrait à partir d'une culture de bactéries Gram-négatif puis purifié selon des techniques classiques, comme décrit par exemple dans Gu & Tsaï, 1993, Infect. Immun. 61 (5) : 1873, Wu et al, 1987, Anal. Biochem.160 : 281 and US 6,531,131. Une préparation de LPS peut être aussi quantifiée en mettant en oeuvre des techniques connues. Une méthode pratique consiste à doser le KDO par chromatographie HPAEC- PAD (high performance anion exchange chromatography).

### Conjugué LPS

Lorsque l'on mélange la TbpB avec un LPS conjugué, le LPS peut être conjugué à n'importe quel oligopeptide ou n'importe quelle protéine porteuse en usage dans le domaine des vaccins ; et notamment l'anatoxine *pertussis,* diphtérique ou tétanique, le mutant de la toxine diphtérique dénommé CRM 197, une OMP bactérienne (par exemple l'OMPC (outer-membrane protein C) de *N. meningitidis*), l'exotoxine A de *Pseudomonas,* la lipoprotéine D d'*Haemophilus influenzae*, la pneumolysine de *Streptococcus pneumoniae* et l'hémagglutinine filamenteuse de *Bordetella pertussis.*

De nombreuses méthodes de conjugaison existent dans le domaine technique. Certaines sont répertoriées par exemple dans les demandes de brevet EP 941 738 et WO 98/31393. Des modes de conjugaison avantageux sont décrits dans une section postérieure relative aux conjugués LPS-TbpB. Ces modes de conjugaison sont pareillement utiles en vue d'obtenir les conjugués envisagés dans la section présente.

### Détoxification du LPS

Lorsque le LPS est simplement purifié ou sous forme de conjugué, il convient de le détoxifier substantiellement avant mélange (ou avant conjugaison puis mélange). La toxicité du LPS est due à son lipide A. Néanmoins, il n'est pas impératif de retirer le lipide A dans son intégralité. En effet, la toxicité étant plus particulièrement liée à une conformation supra moléculaire conférée par la totalité des chaînes d'acides gras portées par le noyau disaccharidique du lipide, selon un mode avantageux, il suffit d'agir au niveau de ces chaînes.

La détoxification peut être obtenue selon des approches diverses : chimique, enzymatique, génétique ou bien encore par complexation avec un peptide analogue de la polymixine B.

L'approche chimique consiste à traiter le LPS par un agent chimique. Selon un mode particulier, le LPS est soumis à une hydrolyse acide douce avec de l'acide acétique qui élimine le lipide A ainsi que les ou les KDO en ramification quand celui-ci (ceux-ci) est (sont) présent(s) dans la structure du LPS. Un tel traitement est par exemple décrit dans Gu & Tsai Infect. Immun. (1993) 61 : 1873. Selon un mode alternatif et préféré, le LPS est soumis à une dé-O-acylation, de préférence primaire, i.a. par traitement à l'hydrazine qui hydrolyse les chaînes d'acides gras primaires estérifiées du lipide A. Un tel traitement est par exemple décrit dans USP 6,531,131, Gupta et al, Infect. Immun. (1992) 60 (8) : 3201 et Gu et al, Infect. Immun. (1996) 64 (10) : 4047.

L'approche enzymatique consiste à mettre le LPS en présence de lipases capables de digérer les chaînes d'acides gras estérifiées du lipide A. De telles lipases sont produites par l'amibe *Dictyostelium discoideum.* Selon un mode particulièrement avantageux, on cultive ensemble (co-culture) l'amibe et une bactérie à Gram-négatif pouvant être phagocytée par l'amibe, telle que *N. meningitidis.* Puis on récupère le surnageant et on en extrait le LPS est alors dépourvu des chaînes d'acides gras. Ce peut être également une acyloxyacyl hydrolase produite par certaines cellules humaines (brevet WO 87/07297 Munford R.) ou par *Salmonella typhimurium* (Trent et al 2001 J. Biol. Chem. 276: 9083-9092; Reynolds et al. 2006 J. Biol. Chem. 281 : 21974-21987) (enzyme codée par les gènes *PagL* ou *LpxR* dans ce dernier cas).

L'approche génétique consiste à utiliser un LPS produit par une souche bactérienne dont le génotype est tel que l'entité du LPS normalement responsable de sa toxicité (le lipide A et plus particulièrement les queues lipidiques du lipide A) présente un degré de toxicité largement réduit ou même inexistant. Une telle souche bactérienne peut être commodément obtenue par mutation. A partir d'une souche sauvage (c'est-à-dire produisant un LPS toxique), il s'agit alors d'inactiver par mutation certains gènes intervenant dans la biosynthèse des chaînes d'acides gras ou dans leur accrochage sur le noyau disaccharidique du lipide A. Ainsi, on peut prévoir d'inactiver les gènes *lpxL*1 ou *lpxL*2 (aussi appelés *htr*B1 / *htr*B2) de *N. meningitidis* ou leur équivalents dans d'autres espèces (par exemple, les équivalent des gènes *lpx*L1 et *lpx*L2 du méningoccoque sont appelés respectivement appelés *msb*B ou *lpx*M et *htr*B ou *lpx*L chez *E. coli*.). Une mutation inactivant l'un de ces gènes conduit à un LPS dépourvu d'une ou de deux chaînes acyles secondaires. Des mutants *lpx*L1 ou L2 de *N. meningitidis* ou d'*Haemophilus influenzae* sont en particulier décrits dans les demandes de brevet WO 00/26384, US 2004/0171133 et WO 97/019688. Chez *N. meningitidis* le gène endogène *lPx*A peut être aussi remplacé par le gène homologue en provenance d'*E. coli* ou *Pseudomonas aeruginosa.* Les chaînes d'acides gras en sont modifiées, avec pour conséquence un lipide A moins toxique (Steeghs et al, Cell. Microbiol. (2002) 4 (9) : 599).

Enfin, une quatrième approche consiste à complexer le LPS avec un peptide analogue de la polymixine B, comme cela est par exemple décrit dans la demande de brevet WO 06/108586. Le LPS complexé et par conséquent, détoxifié est appelé endotoxoïde.

### LPS en liposomes

Lorsque le LPS est formulé en liposomes, il n'apparaît pas forcément nécessaire de le détoxifier au préalable. En effet, le LPS en liposomes - c'est-à-dire associé à la bicouche lipidique formant les liposomes - peut voir sa toxicité décroître de manière très substantielle. L'ampleur de cette décroissance pouvant aller jusqu'à une perte substantielle, est en partie fonction de la nature des composants formant le liposome. Ainsi, lorsque l'on utilise des composants chargés positivement (composants de nature cationique), la perte de toxicité peut être plus importante qu'avec dés composants non-chargés (neutre) ou anioniques.

Par « liposomes », on entend une entité synthétique, de préférence une vésicule synthétique, formée d'au moins une membrane (ou matrice) lipidique bi-couche refermée sur un compartiment aqueux. Aux fins de la présente invention, les liposomes peuvent être unilamellaires (une seule membrane bi-couche) ou plurilamellaires (plusieurs membranes disposées en oignon). Les lipides constituant la membrane bi-couche comportent une région non-polaire qui de manière typique est faite de chaîne(s) d'acides gras ou de cholestérol, et d'une région polaire, typiquement faite d'un groupement phosphate et/ou de sels d'ammonium tertiaires ou quaternaires. En fonction de sa composition, la région polaire peut, notamment à pH physiologique (pH ≈ 7), être porteuse d'une charge de surface nette (globale) soit négative (lipide anionique), soit positive (lipide cationique) ou ne pas porter de charge nette (lipide neutre).

Aux fins de la présente invention, les liposomes peuvent être n'importe quel type de liposomes ; en particulier, ils peuvent être constitués de n'importe quel lipide connu pour son utilité dans la fabrication des liposomes. Le ou les lipides entrant dans la composition des liposomes peuvent être des lipides neutres, anioniques ou cationiques ; ces derniers étant préférés. Ces lipides peuvent être d'origine naturelle (produits d'extraction de plante ou d'oeuf, par exemple) ou synthétique ; ces derniers étant préférés. Les liposomes peuvent être aussi constitués d'un mélange de ces lipides ; par exemple d'un lipide cationique ou anionique et d'un lipide neutre, en mélange. Dans ces deux derniers cas, le lipide neutre est souvent désigné sous le terme de co-lipide. Selon un mode de mélange avantageux, le rapport molaire lipide chargé (cationique ou anionique) : lipide neutre est compris entre 10 : 1 et 1 : 10, avantageusement entre 4 : 1 et 1 : 4, de préférence entre 3 : 1 à 1 : 3, bornes incluses.

En ce qui concerne les lipides neutres, on cite à titre d'exemple : (i) le cholestérol ; (ii) les phosphatidylcholines comme par exemple les 1,2-diacyl-*sn*-glycéro-3-phospho-cholines *e.g*., la 1,2-dioleoyl-*sn*-glycéro-3-phosphocholine (DOPC), ainsi que les 1-acyl-2-acyl-sn-glycéro-3-phosphocholines dont les chaînes acyles sont différentes entres elles (chaînes acyles mixtes) ; et (iii) les phosphatidyléthanolamines comme par exemple les 1,2-diacyl-*sn*-glycéro-3-phosphoéthanolamines *e.g.,* la 1,2-dioleoyl-*sn-*glycéro-3-phosphoéthanolamine (DOPE), ainsi que les 1-acyl-2-acyl-*sn*-glycéro-3-phosphoéthanolamine portant des chaînes acyles mixtes.

En ce qui concerne les lipides anioniques, on cite à titre d'exemple (i) l'hémisuccinate de cholestérol (CHEMS) ; (ii) les phosphatidylsérines telles que les 1,2-diacyl-*sn-*glycéro-3-[phospho-L-sérine] *e.g.,* la 1,2-dioleoyl-*sn*-glycéro-3-[phospho-L-sérine] (DOPS), et les 1-acyl-2-acyl-*sn*-glycéro-3-[phospho-L-sérine] portant des chaînes acyles mixtes ; (iii) les phosphatidylglycérols tels que les 1,2-diacyl-*sn*-glycéro-3-[phospho-*rac*-(1-glycérol)] *e.g.,* la 1,2-dioleoyi-sn-glycéro-3-[phospho-*rac*-(1-glycérol)] (DOPG), et les 1-acyl-2-acyl-*sn*-glycéro-3-[phospho-*rac*-(1-glycérol)] portant des chaînes acyles mixtes ; (iv) les acides phosphatidiques tels que les 1,2-diacyl-*sn*-glycéro-3-phosphate *e.g*., le 1,2-dioleoyl-*sn*-glycéro-3-phosphate (DOPA), et les 1-acyl-2-acyl-*sn*-glycéro-3-phosphate portant des chaînes acyles mixtes ; et (v) les phosphatidylinositols tels que les 1,2-diacyl-*sn*-glycéro-3-(phospho-inositol) *e.g.,* le 1,2-dioleoyl-*sn*-glycéro-3-(phospho-inositol) (DOPI) et les 1-acyl-2-acyl-*sn*-glycéro-3-(phospho-inositol) portant des chaînes acyles mixtes.

En ce qui concerne les lipides cationiques, on cite à titre d'exemple :
(i) les amines lipophiles ou alkylamines comme par exemple le diméthyldioctadécylammonium (DDA), le triméthyldioactadecylammonium (DTA) ou les homologues structuraux du DDA et du DTA [ces alkylamines sont avantageusement utilisées sous forme de sel ; on cite par exemple le bromure de diméthyldioctadécylammonium (DDAB)] ;
(ii) l'octadécenoyloxy(éthyl-2-heptadécenyl-3-hydroxyéthyl) imidazolinium (DOTIM) et ses homologues structuraux ;
(iii) les lipospermines telles que la *N*-palmitoyl-D-erythrospingosyl-1-*O*-carbamoyl spermine (CCS) et la dioctadecylamidoglycylspermine (DOGS, Transfectam) ;
(iv) les lipides incorporant une structure éthylphosphocholine tels les dérivés cationiques des phospholipides, en particulier les dérivés ester phosphoriques de la phosphatidylcholine, par exemple ceux décrits dans la demande de brevet WO 05/049080 et incluant notamment :
   - la 1,2-dimyristoyl-*sn*-glycéro-3-éthylphosphocholine,
   - la 1,2-dipalmitoyl-*sn*-glycéro-3-éthylphosphocholine,
   - la 1,2-palmitoyl-oleoyl-*sn*-glycéro-3-éthylphosphocholine,
   - la 1,2-distearoyl-*sn*-glycéro-3-éthylphosphocholine (DSPC),
   - la 1,2-dioleyl-*sn*-glycéro-3-éthylphosphocholine (DOEPC ou EDOPC ou ethyl-DOPC ou ethyl PC),
   - ainsi que leurs homologues structuraux ;
(v) les lipides incorporant une structure triméthyl ammonium tels que le *N*-(1-[2,3-dioleyloxy]propyl)-*N*,*N*,*N*-trimethylammonium (DOTMA) et ses homologues structuraux et ceux incorporant une structure triméthylammonium propane, tels que le 1,2-dioleyl-3-triméthylammonium propane (DOTAP) et ses homologues structuraux ; ainsi que les lipides incorporant une structure diméthylammonium tels que le 1,2-dioleyl-3-diméthylammonium propane (DODAP) et ses homologues structuraux ; et
(vi) les dérivés cationiques du cholestérol tels que le 3β-[N-(N',N'-dimethylaminoethane)-carbamoyl] cholestérol (DC-Chol) ou d'autres dérivés cationiques du cholestérol comme ceux décrits dans le brevet US 5 283 185 et notamment l'iodure de cholestéryl-3β-carboxamidoéthylènetriméthylammonium, la cholestéryl-3β-carboxyamidoéthylènamine, l'iodure de cholestéryl-3β-oxysuccinamido-éthylènetriméthylammonium et le 3β-[N-(polyéthylénimine)-carbamoyl]-cholestérol.

Par «homologues structuraux », on signifie les lipides qui possèdent la structure caractéristique du lipide de référence tout en s'en différenciant par des modifications secondaires, notamment au niveau de la région non-polaire, en particulier du nombre d'atomes de carbone et des doubles liaisons des chaînes d'acides gras.

Ces acides gras, que l'on trouve aussi dans les phospholipides neutres et anioniques, sont par exemple l'acide dodécanoique ou laurique (C12 :0), l'acide tétradécanoique ou myristique (C14:0), l'acide hexadécanoique ou palmitique (C16:0), l'acide cis-9-hexadecanoique ou palmitoleique (C16:1), l'acide octadécanoique ou stéarique (182 :0), l'acide cis-9-octadécanoique ou oleique (C18:1), l'acide cis,cis-9,12-octadécadiénoiquec ou linoléique (C18:2), l'acide cis-cis-6,9-octadécadiénoique (C18 :2), l'acide ail cis-9,12,15-octadécatriénoique ou α-linolénique (C18 :3), l'acide all cis-6,9,12-octadécatriénoique ou γ-linolénique (C18 :3), l'acide eicosanoique ou arachidique (C20 :0), l'acide cis-9-eicosénoique ou gadoleique (C20:1), l'acide all cis-8,11,14-eicosatrienoique (C20:3), l'acide all cis-5,8,11,14-eicosatetraenoique ou arachidonique (C20 :4), l'acide all cis-5,8,11,14,17-eicosapentaneoique (C20 :5), l'acide docosanoique ou behenique (C22:0), l'acide all cis-7,10,13,16,19-docosapentaenoique (C22:5), l'acide all cis-4,7,10,13,16,19-docosahexaenoique (C22 :6) et l'acide tétracosanoique ou lignocérique (C24 :0).

La structure caractéristique du DDAB est :

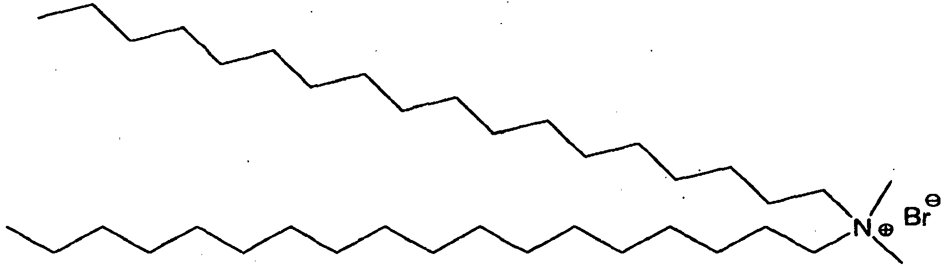

La structure caractéristique de l'ethyl-DOPC est :

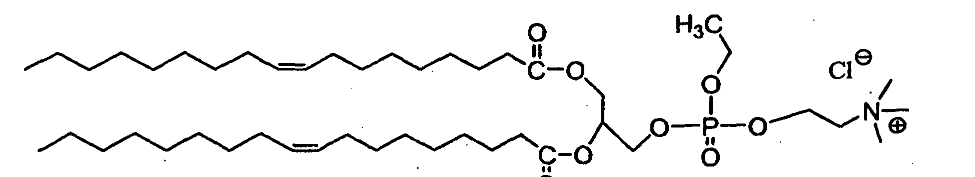

La structure caractéristique du DOTAP est :

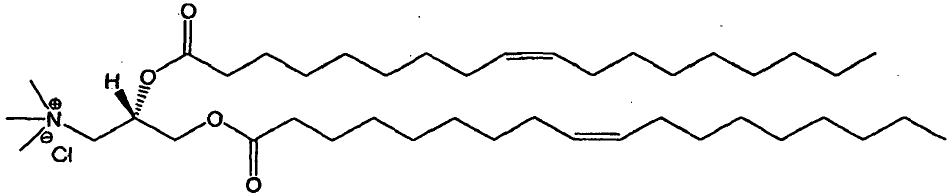

La structure caractéristique du DC-chol est :

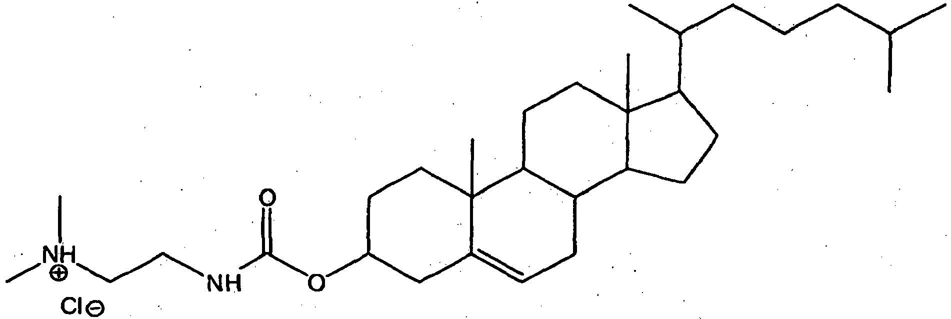

Selon un mode particulier, on utilise un mélange de lipide cationique et de lipide neutre. A titre d'exemple, on cite :
- un mélange de DC-chol et de DOPE, notamment dans un rapport molaire DC-chol : DOPE allant de 10 : 1 à 1 : 10, avantageusement de 4 : 1 à 1 : 4, de préférence d'environ 3 : 1 à 1 : 3;
- un mélange d'ethyl-DOPC et de cholestérol, notamment dans un rapport molaire ethyl-DOPC : cholestérol allant de 10 : 1 à 1 : 10, avantageusement de 4 : 1 à 1 : 4, de préférence d'environ 3 : 1 à 1 : 3; et
- un mélange d'ethyl-DOPC et de DOPE, notamment dans un rapport molaire ethyl-DOPC : DOPE allant de 10: 1 à 1: 10, avantageusement de 4: 1 à 1 : 4 de préférence d'environ de 3 : 1 à 1 : 3.

Selon un mode de préparation avantageux, on réalise dans un premier temps, un film lipidique sec avec tous les composés entrant dans la composition des liposomes. Puis on reconstitue le film lipidique en milieu aqueux, en présence de LPS, par exemple dans un rapport molaire lipides : LPS de 100 à 500, de manière avantageuse de 100 à 400 ; de préférence de 200 à 300 ; de manière tout particulièrement préférée, de 250 environ. D'une manière générale, on estime que ce même rapport molaire ne devrait pas varier de manière substantielle en final du procédé de préparation des liposomes LPS.

De manière générale, l'étape de reconstitution en milieu aqueux conduit à la formation spontanée de vésicules multi-lamellaires dont la taille est ensuite homogénéisée par diminution progressive du nombre de lamelles par extrusion, par exemple à l'aide d'un extrudeur par passages de la suspension lipidique sous pression d'azote, à travers des membranes en polycarbonates de diamètres de pores de plus en plus réduits (0.8, 0.4, 0.2 µm). On peut aussi remplacer le procédé d'extrusion par un autre procédé mettant en oeuvre un détergent (tensio-actif) qui disperse les lipides. Ce détergent est ensuite éliminé par dialyse ou par adsorption sur des microbilles de polystyrène poreuses particulièrement affines de détergent (BioBeads). Quand le tensio-actif se retire de la dispersion lipidique, les lipides se réorganisent en double couche.

A l'issue de l'incorporation du LPS en liposomes, on peut communément obtenir un mélange constitué de liposomes *adhoc* et de LPS forme libre. Avantageusement, les liposomes sont alors purifiés afin de se débarrasser du LPS sous forme libre.

Le LPS ou les liposomes LPS sont finalement mélangés à la TbpB lipidée dans un rapport molaire LPS : TbpB lipidée de 10⁻² à 10³, de manière avantageuse de 10⁻¹ à 10² ; de préférence de 1 à 50 ; de manière tout particulièrement préférée, de 15 à 30 ou de 20 environ.

A titre d'exemple, lorsqu'on utilise du LPS de *N. meningitidis* d'immunotype L8 et la TbpB lipidée de *N. meningitidis,* le rapport molaire peut être typiquement de 20 ou de 25 environ, selon que l'isotype de la TbpB est I ou II. Lorsqu'on utilise du LPS de *N. meningitidis* d'immunotype L6 et la TbpB lipidée de *N. meningitidis,* le rapport molaire peut être typiquement de 19 ou de 23 environ, selon que l'isotype de la TbpB est I ou II.

La TbpB lipidée que l'on utilise pour réaliser ce mélange peut être simplement purifiée ou bien encore incorporée en liposomes ; le premier mode de réalisation étant toutefois préféré.

En raison de sa queue lipidique, on s'attend à ce que, sous forme purifiée, la TbpB lipidée et purifiée présente un certain degré d'insolubilité en condition purement aqueuse. En conséquence, il convient de la placer dans des conditions favorisant sa solubilité. L'homme de l'art maîtrise les techniques visant à rendre soluble une lipoprotéine. Il est par exemple possible d'utiliser un détergent lors de la purification de la lipoprotéine, afin d'obtenir une préparation d'une lipoprotéine purifié soluble en présence de détergent. La quantité de détergent restant dans la préparation finale sera contrôlée de manière à ce qu'elle soit juste nécessaire au maintien sous forme soluble, de la lipoprotéine purifiée. De manière alternative, il est possible d'enlever complètement le détergent utilisé lors de la purification puis de rajouter un autre produit ayant aussi la capacité de maintenir sous forme soluble, la lipoprotéine purifiée.

Sous un deuxième aspect du procédé selon l'invention, on formule ensemble en liposomes (protéoliposomes), le LPS et la TbpB lipidée. Les liposomes utiles à cette fin sont les mêmes que ceux précédemment décrits pour la formulation du seul LPS. Un moyen de mener à bien cette formulation consiste à formuler ensemble en liposomes, le LPS et la TbpB lipidée, par exemple en reconstituant un film lipidique en milieu aqueux en présence de LPS et de TbpB lipidée, notamment dans :
- un rapport molaire lipides : LPS de 100 à 500, de manière avantageuse de 100 à 400 ; de préférence de 200 à 300 ; de manière tout particulièrement préférée, de 250 environ ; et / ou
- un rapport molaire LPS : TbpB lipidée de 10⁻² à 10³, de manière avantageuse de 10⁻¹ à 10² ; de préférence de 1 à 50 ; de manière tout particulièrement préférée, de 15 à 30, *e.g.,* de 20 environ.

A l'issue de l'incorporation en liposomes, du LPS et de la TbpB lipidée, on peut communément obtenir un mélange constitué de liposomes *adhoc* (protéoliposomes), de LPS et/ou de TbpB lipidée sous forme libre. Avantageusement, les liposomes sont alors purifiés afin de se débarrasser du LPS sous forme libre. Une fois le LPS libre éliminé, on peut utiliser le mélange tel quel à des fins vaccinales ou bien les liposomes peuvent être purifiés plus encore afin de se débarrasser de la Tbpb lipidée libre. Une fois les liposomes complètement purifiés, on peut prévoir de rajouter de la Tbpb lipidée libre, notamment en quantité définie.

Sous un mode particulier, les liposomes [LPS] ou les protéoliposomes [LPS + TbpB lipidée] ne contiennent pas de polypeptide autre que la TbpB servant à adjuver le LPS. Par exemple, il peut être intéressant que les liposomes ne contiennent pas de protéines de structure de la membrane externe de bactéries à Gram-négatif telles que les OMP ou les porines ou qu'ils ne contiennent pas de protéine de structure de la membrane externe de la bactérie dont a été extrait le LPS.

Sous un autre mode particulier, un mélange obtenu selon le procédé faisant l'objet de la présente demande ne contient pas de protéines de structure de la membrane externe de bactéries à Gram-négatif telles que les OMPs ou les porines ou ne contient pas de protéines de structure de la membrane externe de la bactérie dont a été extrait le LPS.

### Les conjugués LPS-TbpB lipidée

Sous un troisième aspect du procédé selon l'invention, on conjugue le LPS avec la TbpB lipidée. De manière avantageuse, il convient de détoxifier substantiellement le LPS avant conjugaison. La détoxification peut être menée à bien comme décrit précédemment, de manière chimique, enzymatique, génétique ou par complexation.

De nombreuses méthodes de conjugaison existent dans le domaine technique. Certaines sont répertoriées par exemple dans les demandes de brevet EP 941 738 et WO 98/31393.

D'une manière générale, les groupements réactifs du LPS mis en jeu lors de la conjugaison sont ceux du core interne (inner core) ou du lipide A. Il peut s'agir entre autre de la fonction acide du KDO ou bien d'un aldéhyde généré suite à un traitement approprié sur le disaccharide du lipide A. Par exemple, un traitement à la phosphatase génère un aldéhyde sur la structure de la deuxième glucosamine du lipide A de *N. meningitidis* (Brade H. (2002) J. Endotoxin Res. 8 (4) : 295 Mieszala et al, (2003) Carbohydrate Res. 338 : 167 et Cox et al, (2005) Vaccine 23 (5) : 5054).

De manière avantageuse, le procédé de conjugaison fait usage (i) d'un agent de liaison bifonctionnel (linker) ou (ii) d'un espaceur et d'un linker.

Par exemple, dans le premier cas, on active le LPS avec un agent de couplage bifonctionnel (linker) de formule R1 - A - R2 de telle sorte que le radical R2 réagisse avec un groupement réactif du KDO ou du lipide A afin d'obtenir un LPS activé ; puis on conjugue le LPS activé avec la TbpB lipidée de telle sorte que le radical R1 réagisse avec un groupement fonctionnel porté par la TbpB lipidée afin d'obtenir un conjugué.

Par exemple, dans le deuxième cas, on dérive le LPS avec un espaceur de formule R3 - B - R4 de telle sorte que le radical R3 réagisse avec un groupement réactif du KDO ou du lipide A afin d'obtenir un LPS dérivé ; puis on active le LPS dérivé avec un agent de couplage bifonctionnel (linker) de formule R1 - A - R2 de telle sorte que le radical R2 réagisse avec le radical R4 afin d'obtenir un LPS dérivé et activé ; enfin on conjugue le LPS dérivé et activé avec la TbpB lipidée, de telle sorte que le radical R1 réagisse avec un groupement fonctionnel porté par la TbpB lipidée afin d'obtenir un conjugué.

Dans le deuxième cas on peut aussi procéder de la manière suivante : On dérive la lipoprotéine avec un espaceur de formule R3 - B - R4 de telle sorte que le radical R4 réagisse avec un groupement fonctionnel porté par la TbpB lipidée ; on active le LPS avec un agent bifonctionnel (linker) de formule R1 - A - R2 de telle sorte que le radical R2 réagissent avec un groupement réactif du KDO ou du lipide A afin d'obtenir un LPS activé ; puis, on conjugué le LPS activé avec la TbpB dérivée de telle sorte que le radical R1 du LPS activé réagisse avec le radical R3 de la TbpB dérivée afin d'obtenir un conjugué.

Dans la formule de l'espaceur, B peut être une chaîne carbone, de préférence carbonyl, alkyl ou alkylène, par exemple en C1 à C12. R3 et R4 peuvent être de manière indépendante un groupe thiol ou amine ou un résidu le portant, par exemple un groupe hydrazide *i*.*e*., NH₂-NH-CO-. Des composés pouvant être utilisés comme espaceur sont par exemple de formule NH₂ - B -NH2, ou de préférence NH₂ - B - SH et NH₂ - B - S - S - B' - NH₂. A titre d'exemple particulier on cite : la cystéamine, la cystéine les diamines *e.g*., le diaminohexane, l'acide adipique dihydrazide (ADH) l'urée et la cystamine.

Dans la formule du linker, A peut être une chaîne aromatique ou de préférence aliphatique substituée ou non, qui de manière avantageuse, comprend de 1 à 12 atomes de carbone ; de préférence 3 à 8 atomes de carbone. Par exemple A peut être un C2 à C8 alkylène, un phénylène, un C7 à C12 aralkylène, un C2 à C8 alkyle, un phényle, un C7 à C12 aralkyle, C6 alkanoyloxy ou un benzylcarbonyloxy, substitué ou non.

Le radical R2 est le groupement fonctionnel du linker qui crée le lien au LPS ou au LPS dérivé. Ainsi, R2 est un groupement fonctionnel qui peut réagir avec un groupe carboxyle, hydroxyle, aldéhyde ou amine. Si le linker doit réagir avec un groupe hydroxyle carboxyle ou aldéhyde, R2 est de préférence un groupe amine ou un résidu le portant, par exemple un groupe hydrazide *i.e.,* NH₂-NH-CO-. Si le linker doit réagir avec un groupe aminé, R2 est de préférence un groupe carboxyl, succinimidyl (*e.g*., N-hydroxy succinimidyl) ou sulfosuccinimidyl (*e*.*g*., N-hydroxy sulfosuccinimidyl).

Ainsi, des composés pouvant être utilisés comme linker peuvent être choisis parmi l'acide adipique dihydrazide (ADH) ; le sulfosuccinimidyl-6-(3-[2-pyridyldithio] propionamido)-hexanoate (Sulfo-LC-SPDP) ; le succinimidyl-6-(3-[2-pyridyldithio] propionamido)-hexanoate (LC-SPDP) ; le N-succinimidyl-S-acetyl thioacetate (SATA) ; le N-Succinimidyl-3-(2-pyridyl dithio) propionate (SPDP), succinimidyl acetyl thiopiopionate (SATP) ; le succinimidyl-4-(N-maleimido methyl) cyclohexane-1-carboxylate (SMCC) ; le maleimide benzoyl-N-hydroxy succinimide ester (MBS) ; le N-succinimidyl (4-iodoacetyl) aminobenzoate (SIAB) ; le succinimidyl 4-(p-maleimidophényl) butyrate (SMPB) ; le bromoacétique acide -N-hydroxy succinimide (BANS) ester ; le dithiobis (succinimidyl propionate) (DTSSP); le H-(γ-maléimido butyryloxy) succinimide ester (GMBS) ; le succinimidyl-4-(N-maléimidométhyl) cyclohexane-1-carboxylate ; le N-succinimidyl-4-(4-maléimidophényl) butyrate ; le *N-*[ß-maleimidocaproic acid] hydrazide (BMCH) ; le N-succinimidyl-4-maléimido-butyrate ; et le N-succinimidyl-3-maléiimido-benzoate.

A titre d'exemple, on propose d'utiliser la fonction acide du KDO pour dériver le LPS avec de l'ADH en présence d'un carbodiimide [*e*.*g*., 3-diméthylaminopropyl)-3-ethyle carbodiimide hydrochloride (EDAC)]. Puis on fait réagir la fonction amine ainsi introduite avec les fonctions carboxyles dde la TbpB, en présence d'EDAC, après avoir protégé les fonctions amines de ce dernier (Wu et al (2005) Vaccine 23 : 5177) ou les avoir transformées en fonctions acide (Succinylation de la protéine ; Pavliakova et al, Infect. Immun. (1999) 67 (10) : 5526).

De manière alternative, on propose d'utiliser la fonction acide du KDO pour dériver le LPS avec de la cystéamine ou de la cystéine en présence d'EDAC. Puis on fait réagir la fonction thiol ainsi introduite avec la fonction maleimide d'un linker homobifonctionnel, tel que le bis maleimido hexane ; ou hétérobifonctionnel, tel que le GMBS. Dans le premier cas, on fait alors réagir la fonction maleimide ainsi introduite avec les fonctions thiols de la TbpB. Dans le deuxième cas, on fait réagir la fonction succinimidyle du LPS dérivé et activé avec les fonctions amines de la TbpB.

En fonction du mode de conjugaison retenu ; le LPS et la TbpB lipidée sont conjugués l'un à l'autre dans un rapport molaire LPS : TbpB lipidée de 10⁻¹ à 10², de manière avantageuse de 1 à 10², de préférence de 1 à 50 ; de manière tout particulièrement préférée de 20 environ.

Sous un autre aspect, l'invention a aussi pour objet un vaccin qui ne contient pas d'OMVs et qui comprend (i) un antigène vaccinal qui est le LPS d'une bactérie à Gram-négatif, optionnellement formulé en liposomes ou conjugué à un polypeptide porteur ou sous forme d'endotoxoïde, et (ii) un adjuvant de la réponse immune anti-LPS qui est la TbpB lipidée de *N. meningitidis*.

On comprend aisément que le vaccin selon l'invention ne contient pas non plus de protéosomes (mélange de protéines membranaires indéfini) directement préparés à partir de leur origine bactérienne naturelle. Ainsi, les porines telles que PorA et PorB, qui sont des protéines majeures de la membrane externe des bactéries à Gram-négatif, sont absentes, *i.a.* sous leur forme non-recombinante, de préférence définitivement absentes sous quelque forme que ce soit.

Un vaccin selon l'invention peut se décliner selon des modes variés. Ainsi, un vaccin selon l'invention peut comprendre :
A - (i) un antigène vaccinal qui est le LPS d'une bactérie à Gram-négatif et (ii) un adjuvant de la réponse immune anti-LPS qui est la TbpB lipidée de *N. meningitidis ;* le LPS étant formulé en liposomes ;
B - (i) un antigène vaccinal qui est le LPS d'une bactérie à Gram-négatif et (ii) un adjuvant de la réponse immune anti-LPS qui est la TbpB lipidée de *N. meningitidis ;* le LPS et la TbpB étant formulés ensemble en liposomes ;
C - (i) un antigène vaccinal qui est le LPS d'une bactérie à Gram-négatif et (ii) un adjuvant de la réponse immune anti-LPS qui est la TbpB lipidée de *N. meningitidis ;* le LPS étant sous forme d'endotoxoïde (c'est-à-dire complexé à un peptide analogue de la polymixine B) ;
D - (i) un antigène vaccinal qui est le LPS d'une bactérie à Gram-négatif et (ii) un adjuvant de la réponse immune anti-LPS qui est la TbpB lipidée de *N. meningitidis ;* le LPS étant conjugué à un polypeptide porteur ; ou
E - (i) un antigène vaccinal qui est le LPS d'une bactérie à Gram-négatif et (ii) un adjuvant de la réponse immune anti-LPS qui est la TbpB lipidée de *N. meningitidis ;* le LPS étant conjugué à la TbpB.

Une composition vaccinale selon l'invention est notamment utile pour traiter ou prévenir une infection à bactérie à Gram-négatif non-entérique (telles que les bactéries des genres *Neisseriae, Bordetellae, Branhamellas, Haemophilus* et *Moraxellae) ;* ou des genres *Klebsiella, Pseudomonas, Burkolderia, Porphyromonas, Franciscella, Yersinia, Enterobacter, Salmonella, Shigella, Escherichia e.g, E. coli.*

Sous un aspect préféré une composition vaccinale selon l'invention est notamment utile pour traiter ou prévenir une infection à *N*. *meningitidis,* telles que les méningites à *N. meningitidis,* les méningococcémies et les complications qui peuvent en dériver telles que le *purpura fulminans* et le choc septique ; ainsi que les arthrites et péricardites à *N. meningitidis.*

Elle peut être fabriquée de manière conventionnelle. En particulier, on associe une quantité efficace d'un point de vue thérapeutique ou prophylactique des constituants du vaccin que sont le LPS et la TbpB lipidée, à un support ou à un diluant.

Un vaccin selon l'invention peut comprendre un ou plusieurs TbpBs lipidées. En outre, il peut comprendre un ou plusieurs antigène(s) vaccinal(aux) additionnel(s). De manière avantageuse, ils peuvent être choisis parmi les protéines de l'espèce bactérienne d'où provient le LPS. En lien avec cette possibilité, une composition vaccinale selon l'invention peut en outre contenir un adjuvant additionnel, acceptable d'un point de vue pharmaceutique, destiné à adjuver le ou les antigène(s) vaccinal(aux) additionnel(s) ; néanmoins cette option n'est pas la plus attrayante en raison de sa complexité.

C'est pourquoi, selon un mode préféré, le vaccin selon l'invention ne contient pas de composé ayant une fonction d'adjuvant autre que le LPS et la TbpB.

Les quantités de LPS et de TbpB lipidée par dose de vaccin qui sont suffisantes pour atteindre les fins pré-citées, efficaces d'un point de vue immunogène, prophylactique ou thérapeutique, dépendent de certains paramètres incluant l'individu traité (adulte, adolescent, enfant ou nourrisson), la voie d'administration et de sa fréquence.

Ainsi, la quantité de LPS par dose suffisante pour atteindre les fins pré-citées est notamment comprise entre 5 et 500 µg, avantageusement entre 10 et 200 µg, de préférence entre 20 et 100 µg, de manière tout à fait préférée entre 20 et 80 µg ou entre 20 et 60 µg, bornes incluses.

Dans le vaccin selon l'invention, la quantité de TbpB lipidée par dose est comprise entre 5 et 500 µg, avantageusement entre 10 et 200 µg, de préférence entre 20 et 100 µg, de manière tout à fait préférée entre 20 et 80 µg ou entre 20 et 60 µg, bornes incluses.

Dans le vaccin selon l'invention, le rapport molaire LPS : TbpB lipidée est de 10⁻² à 10³, de manière avantageuse de 10⁻¹ à 10² ; de préférence de 1 à 50 ; de manière tout particulièrement préférée, de 15 à 30 ou de 20 environ.

Le terme « dose » employé ci-dessus doit être entendu comme désignant un volume de vaccin administré à un individu en une seule fois - c'est-à-dire à un temps T. Des doses conventionnelles sont de l'ordre du millilitre, par exemple 0.5, 1 ou 1.5 ml ; le choix définitif dépendant de certains paramètres et notamment de l'âge et du statut du receveur. Un individu peut recevoir une dose répartie en plusieurs sites d'injection le même jour. La dose peut être unique ou si nécessaire, l'individu peut aussi recevoir plusieurs doses à un certain délai d'intervalle - délai d'intervalle pouvant être déterminé par l'homme de l'art.

Le LPS et la TbpB lipidée en usage dans le vaccin selon l'invention peuvent respectivement être n'importe quels LPS et TbpB décrits comme pouvant être utilisés dans le procédé selon l'invention. Le LPS et la TbpB peuvent être formulés ou préparés comme décrit en ce qui concerne le procédé selon l'invention.

Elle peut être administrée par n'importe quelle voie conventionnelle en usage dans le domaine de l'art, *e.g*. dans le domaine des vaccins, notamment par voie entérale ou parentérale. L'administration peut avoir lieu en dose unique ou répétée avec un certain délai d'intervalle. La voie d'administration varie en fonction de divers paramètres par exemple de l'individu traité (condition, âge, etc.).

Aussi décrites sont :
- Une méthode pour induire chez un mammifère, par exemple un humain, une réponse immune à l'encontre d'une bactérie pathogène à Gram-négatif, selon laquelle on administre au mammifère une quantité efficace d'un point de vue immunogénique d'un vaccin selon l'invention pour induire une réponse immune, en particulier une réponse immune protectrice à l'encontre de la bactérie pathogène à Gram-négatif ; et
- Une méthode de prévention et/ou de traitement d'une infection induite par une bactérie pathogène à Gram-négatif selon laquelle on administre une quantité efficace d'un point de vue prophylactique ou thérapeutique d'un vaccin selon l'invention à un individu ayant besoin d'un tel traitement.

L'invention est illustrée par la partie expérimentale comme suit.

### Données expérimentales

### 1. Préparation de la rTbpB lipidée

Par souci de simplification langagière, on indiquera simplement par la suite, rTbpB.

### 1.1. Production

### Souche

La souche d'expression est la souche d'*E*. *coli* BL21 contenant le plasmide pTG9219. Ce plasmide comporte notamment un marqueur de sélection à la kanamycine et le polynucléotide codant pour la rTbpB de la souche de *N. meningitidis* M982 dont la séquence est telle que décrite dans le brevet EP 586 266, fusionnée à la séquence signal RIpB (Real lipoprotein B) d'*E*. *coli* et placée sous le contrôle du promoteur arabinose (*ara*B).

### Culture

Trois congelâts de la souche d'*E*. *coli* BL21/pTG9219 (1 ml chacun) servent à ensemencer 3 litres de milieu LB (Luria Broth) répartis en erlens. L'incubation est poursuivie 15 à 18 h à 37°C.

Cette pré-culture sert à ensemencer un fermenteur contenant du milieu TGM16 (extrait de levure 9g/L, K₂SO₄ 0.795 g/L, K₂HPO₄ 3.15 g/L, NaCl 0.75 g/L, CaCl₂,2H₂O 0.005 g/L, FeCl₃,6H₂O 0.021 g/L, MgSO₄,7H₂O 0.69 g/L, hydrolysat acide de caséine sans sel 37.5 g/L) supplémenté avec du glycérol 20 g/L, à raison de 10 % (vol./vol.).

La culture est poursuivie à 37°C sous agitation, à une pression de 100 mbar et sous une alimentation d'air de 1 L/min/L de culture, en réajustant au cours du temps la concentration de glycérol à 20 g/L (*e.g.* à DO₆₀₀ de 15 ± 2). Lorsque la DO₆₀₀ est comprise entre 21 et 27, on induit l'expression de la rTbpB par addition d'arabinose pour obtenir une concentration finale de 10 g/L. Après une heure d'induction, la culture est arrêtée en refroidissant aux alentours de 10°C.

Les culots bactériens sont récupérés par centrifugation et stockés au froid.

### 1.2. Purification

### Extraction des membranes renfermant la rTbpB

### Extraction des LPS

Un culot bactérien équivalent à un litre de culture (environ 72 g de germes, poids humide) est décongelé à une température de 20°C +/-5°C. Les germes décongelés (ou partiellement décongelés) sont remis en suspension par 800 ml d'une solution à température ambiante de Tris HCl 50 mM, EDTA 5 mM pH 8,0. On ajoute aussitôt 9 pastilles d'inhibiteur de protéases (7 pastilles de Complete Mini, EDTA free ; ROCHE réf. 11836170001 + deux pastilles de Complete, EDTA free; ROCHE réf. 11836170001). Une partie des germes se lysant spontanément, 4 µl de benzonase (1 UI d'activité DNAse/ml en final ; Merck réf K32475095) sont ajoutés également. L'incubation est poursuivie à +4°C durant 45 minutes sous agitation magnétique après homogénéisation par Turrax (15 sec.).

Puis on ajoute 4 ml de MgCl₂ 1 M afin d'être à 5 mM final. L'agitation magnétique est portée à 10 minutes. Une centrifugation à 15,000 g durant 45 minutes permet de récolter le culot (culot C1 ; *vs* Surnageant S1) contenant la protéine rTbpB.

Une deuxième extraction est réalisée : homogénéisation par Turrax dans 800 ml du tampon Tris HCl 50 mM, EDTA 5 mM, pH 8,0 et agitation pendant 30 min. On ajoute du MgCl₂ (8 ml d'une solution molaire). L'incubation est poursuivie pendant 10 minutes. La suspension est centrifugée 15,000 g durant 1 hr 30.

### Lyse bactérienne

Le culot est remis en suspension par 1400 ml de Tris HCl 50 mM additionné de 4 pastilles d'inhibiteur de protéases et de 8 µl de Benzonase. La solution est homogénéisée au Turrax 15 secondes. La lyse est réalisée à +4°C durant 30 minutes grâce à l'ajout de 14 ml (10 mg/ml en final) de lysozyme à 100 mg/ml en acétate de Na 25 mM, glycérol 50 %.

La suspension est centrifugée à 30,000 g durant 30 minutes (Culot C2 contenant la protéine ; *vs* surnageant S2 contenant les contaminants de rTbpB). Le culot contenant les membranes peut être congelé à ce stade.

### Lavage des fragments de membranes

Le culot de lyse C2 est repris en Tris HCl 50 mM (1100 ml). Après homogénéisation, (Turrax 15 secondes), il est lavé durant une heure à +4°C. On opère comme précédemment à une centrifugation à 30,000 g durant 30 minutes, Le culot (C3 ; *vs* surnageant S3) est congelé à -45°C. Le tampon Tris HCl 50 mM permet d'éliminer une petite quantité de protéine (surnageant S3) et ne solubilise que très peu de rTbpB.

Le culot C3 est repris en tampon Tris HCl 50 mM, urée 8 M pH 8,0 (800 ml). Ce tampon permet d'éliminer une partie des protéines contaminantes sans solubiliser les membranes contenant la rTbpB. Après homogénéisation (sans Turrax), la solution est alors agitée durant une heure à +4°C. On procède comme précédemment à une centrifugation à 30,000 g durant 30 minutes qui permet d'obtenir un culot de membranes qui peut être congelé.

### Solubilisation des membranes

Le culot de membrane décongelé est solubilisé par 780 ml de tampon Tris HCl 50 mM EDTA 6 mM Urée 2 M Elugent 4 % à pH 7,5. La présence du détergent à 4 % et de l'urée 2 M permet de réaliser la solubilisation du culot. La solution est agitée à +4°C une nuit (16h minimum). La centrifugation à 30,000 g (1 heure à +4°C) de la solution ne laisse qu'un petit culot (C4) contenant quelques impuretés. Le surnageant S4 contenant la protéine rTbpB est récupéré pour dépôt sur une première colonne échangeuse de cations (QS I).

### Purification par chromatographie échangeuse d'anions sur Q Sepharose à pH 7,5

Deux chromatographies successives sont réalisées, le produit de la première chromatographie est collecté puis déposé ensuite après une étape de dialyse sur une deuxième colonne de chromatographie qui utilise des conditions différentes (absence d'EDTA).

### 1^{ère} Chromatographie, en présence d'EDTA (Chromatographie QSI)

Une colonne de 600 ml (K50, diamètre 20 cm2) de gel Q Sepharose Fast Flow (réf 17-0510-01 GE Healthcare) est montée, tassée en tampon d'équilibration Tris HCl 50 mM EDTA 6 mM, Urée 2 M, Elugent 1 %, à pH 7,5 au débit de 8 ml/minute.

Le surnageant S4 (environ 845 ml) est déposé au débit de 6 ml/minute. L'éluât direct (partie qui ne s'accroche pas sur la colonne lors du dépôt de l'échantillon) contient la protéine d'intérêt rTbpB. Cet éluât (1150 mL) est prélevé, puis dialysé à +4°C (durant 6 jours) contre 6 litres de tampon Tris HCl 50 mM, Urée 2 M, Elugent 1 %, pH 7,5 afin d'abaisser à 1 mM la concentration en EDTA et d'éliminer le NaCl.

### 2^{éme} chromatographie (QS II), sans EDTA

Une colonne K50 de 490 ml de gel neuf Q Sepharose Fast Flow est équilibrée en tampon Tris HCl 50 mM, Urée 2 M, Elugent 1 % pH 7,5.

La solution dialysée (1080 ml) est déposée sur la colonne (débit 6 ml/minute) ; puis 5 paliers d'élution salin dans ce même tampon sont réalisés: 20 mM, 50 mM, 100 mM, 250 mM et 1 M NaCl (débit de travail 6 ml/minute). La protéine rTbpB est éluée de la colonne à deux concentrations en sel (50 mM et 100 mM). La fraction d'élution 50 mM est la fraction d'intérêt, la protéine rTbpB y étant la plus pure et en plus importante quantité (2,6 fois plus de protéine que dans la fraction 100 mM de NaCl)

Le pH de la fraction correspondant au pic d'élution 50 mM NaCl est abaissé sous agitation magnétique à pH 5,5 par un ajout d'acide acétique 1,7 N. La solution (860 ml) est placée en dialyse contre 5 litres de tampon acétate de sodium 10 mM, Urée 1 M, Elugent 0,2 %, pH 5,5 (24 hrs à +4°C) puis contre 4 litres de tampon acétate de sodium 10 mM, Urée 1 M, Elugent 0,2 %, pH 5,5 (17 hrs à +4°C).

### Purification par chromatographie échangeuse de cations sur SP Sepharose (SPI) à pH 5,5

Une colonne K50 de 100 ml de gel neuf SP Sepharose Fast Flow (Ge Healthcare, réf 17-0729-01) est équilibrée en tampon acétate de sodium 10 mM, Urée 1 M, Elugent 0,2 %, pH 5,5.

La solution de protéine dialysée (850 ml) est déposée sur la colonne (débit 6 ml/minute). Puis, cinq paliers salins d'élution sont réalisés: 50 mM, 100 mM, 250 mM, 500 mM et 1 M NaCl, en tampon cité ci-dessus.

La protéine rTbpB est éluée exclusivement dans la fraction 250 mM NaCl et les contaminants de faible poids moléculaire sont éliminés essentiellement dans l'éluât direct (40 %). On obtient ainsi environ 35 mg de rTbpB purifiée.

### Dialyse et concentration du produit de SPI (fractions 250 mM)

Les fractions correspondant au pic d'élution 250 mM de la colonne SPI sont rassemblés (volume 274 ml). Le pH de la solution est remonté à pH 7,3 par ajout sous agitation d'environ 800 µl de NaOH 0,5 N. La solution est placée en dialyse à +4°C (Spectra Por 1 : seuil de coupure 6-8000 D) contre deux bains de 10 litres de PBS Elugent 0,2 % pH 7,1 (66 hrs et 22 hrs).

Le dialysat est concentré jusqu'à un volume de 21,1 ml par diafiltration concentration frontale sur membrane Amicon 30 kD en PBS (réf PBTK06510).

Puis le concentré obtenu est remis en dialyse contre 2 litres de PBS Elugent 0,2 % pH 7,1 (Slide A Lyser réf 66810: seuil de coupure 10 kD).

La solution est ensuite filtrée de façon aseptique sur filtre Millex 0,22 µm en Durapore (réf Millipore SLGV 033RS). Le lot de protéine rTbpB purifiée obtenu est congelé à - 80°C. La concentration en protéine est de 1642 µg/ml.

### 1.3. Préparation de la rTbpB pour injection

La solution de rTbpB obtenue en section 1.2. est traitée par adsorption sur des Bio-Beads™ SM-2 pour éliminer l'excès du détergent Elugent™ (tensio-actif constitué d'alkyl glucosides) qui pourrait déstabiliser les liposomes LPS L8.

### Activation des Bio-Beads™

On ajoute environ 2.5 mL de méthanol à 500 mg de Bio-Beads™ et on homogénéise par intermittence durant 15 min. à température ambiante. Après un temps de décantation, le surnageant est éliminé. Cette opération de lavage est renouvelée deux fois.

On ajoute alors environ 5 mL d'eau stérile ultrafiltrée et on homogénéise par intermittence durant 15 min. à température ambiante. Après un temps de décantation, le surnageant est éliminé. Cette opération de lavage est renouvelée deux fois.

On ajoute alors environ 5 mL de PBS et on homogénéise par intermittence durant 15 min. à température ambiante. On conserve à 5°C et on utilise le jour même.

En final, le poids des Bio-Beads™ s'est accru d'un facteur R (égal à environ 1.2).

### Elimination du détergent par adsorption sur Bio-Beads™

La solution de rTbpB obtenue à la section 1.2. contient 2 mg/mL d'Elugent™. La quantité de Bio-Beads™ devant être utilisée est déterminée en fonction de la quantité d'Elugent™ à éliminer.

Pour un mL de la solution de rTbpB obtenue à la section 1.2., on ajoute 29 X *R* mg de Bio-Beads™ activées. On agite vivement pendant une heure à température ambiante. Puis on récupère le maximum de liquide auquel on ajoute du merthiolate 0.001 % final. Le tout est réalisé en condition stérile.

### 2. Préparation du LPS L8 purifié

### Culture

Huit mL de congelât de la souche A1 de *N. meningitidis* sérotype A connue pour exprimer de manière exclusive l'immunotype L8 servent à ensemencer 800 mL de milieu Mueller Hinton (Merck) supplémentés avec 4 mL d'une solution de glucose à 500 g/L et répartis en erlens. La culture est poursuivie sous agitation à 36 ± 1°C pendant environ 10 heures.

On ajoute à la pré-culture 400 mL d'une solution de glucose à 500 g/L et 800 mL d'une solution d'acides aminés. Cette préparation sert à ensemencer un fermenteur contenant du milieu Mueller-Hinton, à une DO₆₀₀ₙₘ proche de 0.05. La fermentation est poursuivie à 36°C, à pH 6.8, 100 rpm, pO₂ 30 % sous un flux d'air initial de 0.75 L/min/L de culture.

Après environ 7 hrs (DO₆₀₀nm d'environ 3), on ajoute du milieu Mueller-Hinton à raison, de 440 g/hr. Quand la concentration en glucose est inférieure à 5 g/L la fermentation est arrêtée. La DO₆₀₀ₙₘ finale est couramment comprise entre 20 et 40. Les cellules sont récoltées par centrifugation et les culots congelés à -35°C.

### Purification

Les culots sont décongelés et suspendus avec 3 volumes de phénol 4.5 % (vol./vol.) en agitant vigoureusement pendant 4 hrs à environ 5°C. Le LPS est extrait par traitement au phénol.

La suspension bactérienne est chauffée à 65°C puis mélangée vol./vol. avec du phénol à 90 % en agitant vigoureusement pendant 50-70 min à 65°C. La suspension est ensuite refroidie à température ambiante puis centrifugée pendant 20 min à 11 000 g. La phase aqueuse est prélevée et conservée tandis que la phase phénolique et l'interphase sont récoltées pour être soumises à une deuxième extraction.

La phase phénolique et l'interphase sont chauffées à 65°C puis mélangées à un volume d'eau équivalent à celui de la phase aqueuse précédemment prélevée en agitant vigoureusement pendant 50-70 min à 65°C. La suspension est ensuite refroidie à température ambiante puis centrifugée pendant 20 min à 11 000 g. La phase aqueuse est prélevée et conservée tandis que la phase phénolique et l'interphase sont récoltées pour être soumises à une troisième extraction identique à la seconde.

Les trois phases aqueuses sont dialysées séparément contre 40 L d'eau chacune. Puis les dialysats sont rassemblés ensemble. A 9 volumes de dialysat on ajoute un volume de Tris 20 mM, MgCl2 2mM. Le pH est ajusté à 8.0 ± 0.2 avec de la soude 4 N.

Deux cent cinquante unités internationales de DNAse sont ajoutées par gramme de culot. Le pH est ajusté à 6.8 ± 0.2. La préparation est placée à 37°C pendant environ 2 hrs sous agitation magnétique ; puis soumise à une filtration sur membrane de 0.22 µm. Le filtrat purifié par passage sur une colonne de Sephacryl S-300 (5.0 x 90 cm ; Pharmacia™).

Les fractions contenant le LPS sont rassemblées ensemble et la concentration en MgCl₂ est élevée à 0.5 M en rajoutant de la poudre de MgCl₂,6H₂O sous agitation.

Tout en poursuivant l'agitation, on ajoute de l'alcool absolu déshydraté pour une concentration finale de 55 % (vol./vol.). L'agitation est poursuivie une nuit à 5 ± 2°C, puis on centrifuge à 5,000 g pendant 30 min à 5 ± 2°C. Les culots sont resuspendus avec au moins 100 mL de MgCl2 0.5 M puis soumis à une deuxième précipitation alcoolique identique à la précédente. Les culots sont resuspendus avec au moins 100 mL de MgCl2 0.5 M.

La suspension est soumise à une filtration sur gel comme précédemment décrit. Les fractions contenant le LPS sont rassemblées ensemble et stérilisées par filtration (0.8-0.22 µm) et conservées à 5 ± 2°C.

Ce procédé de purification permet d'obtenir environ 150 mg de LPS L8 par litre de culture.

### 3. Production d'un endotoxoïde L8 (LPS L8 détoxifié par complexation à un peptide analogue de la polymixine B)

Cet endotoxoïde est préparé comme décrit dans la demande de brevet WO 06/108586. Brièvement, un volume d'une solution de LPS L8 purifié à 1 mg/mL, stérilisée par filtration sur membrane de 0.22 µm, est mélangé à un volume d'une solution de peptide SAEP2-L2 à 1 mg/mL stérilisée par filtration sur membrane de 0.22 µm.

Le peptide SAEP2-L2 est un peptide de structure dimérique antiparallèle, de formule :

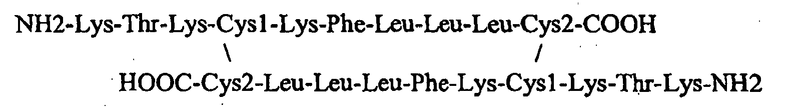

Un précipité se forme immédiatement. On mélange 5 min à température ambiante, puis on laisse reposer une nuit à 4 °C. Le précipité est récolté par centrifugation à 3,000 rpm pendant 10 min. Le culot est lavé 5 fois avec un volume d'eau stérile dépourvue de pyrogénicité, pH 7.2. Enfin, le culot est resuspendu en tampon Tris 10 mM NaCl 150 mM Tween 80, pH 7.4 pour obtenir une suspension à 1 mg/mL, calcul basé sur le poids humide du précipité. La suspension est conservée à 4°C.

### 4. Préparation d'un mélange endotoxoïde L8 + rTbpB

On mélange de la rTbpB en PBS (obtenue tel que décrit dans la section 1.3.) avec de l'endotoxoïde (section 3.) dans un rapport poids : poids égal à 1. Puis on ajuste en tampon Tris 10 mM NaCl 150 mM Tween 80 0,05 % pour obtenir une préparation dans laquelle chacun des composants est concentré à 80 µg/ml.

### 5. Préparation des liposomes [LPS L8] par dialyse de détergent

### 5.1. Préparation des liposomes

Les liposomes LPS L8 sont préparés par dialyse de détergent. En bref, les lipides (EDOPC : DOPE) sont mis sous forme de film lipidique et repris en tampon Tris 10 mM, puis dispersés en présence dé 100 mM d'octyl β-D-glucopyranoside (OG) (Sigma-Aldrich ref 08001) et filtrés stérilement. Le LPS L8 en OG 100 mM est ajouté stérilement. Le mélange lipides/LPS/OG est ensuite dialysé contre du tampon Tris 10 mM pour éliminer l'OG et former les liposomes.

### Protocole

On réalise une préparation lipidique en chloroforme des lipides que l'on va utiliser pour la fabrication des liposomes. Un film sec est obtenu par évaporation complète du chloroforme.

Un film sec de 1,2 dioleyl-*sn*-glycero-3-ethylphosphocholine (EDOPC ou ethyl-DOPC) et de 1,2-dioleoyl-*sn*-glycero-3-phosphoethanolamine (DOPE) dans un rapport molaire EDOPC : DOPE de 3 pour 2 est obtenu en mélangeant 12.633 mL d'une solution de EDOPC (Avanti Polar Lipids ref 890704) à 20 mg/ml en chloroforme et 7.367 mL d'une solution de DOPE (Avanti Polar Lipids ref 850725) à 20 mg/ml en chloroforme et en évaporant le chloroforme jusqu'à disparition complète.

Le film sec est repris par 30 mL de tampon Tris 10 mM pH 7.0 pour obtenir une suspension contenant 13.333 mg de lipides /ml (8.42 mg/ml d'EDOPC et 4.91 mg/ml de DOPE). La suspension est agitée 1 hr à température ambiante puis soniquée 5 min dans un bain.

Puis on ajoute toujours sous agitation, 3.333 ml d'une solution stérile d'octyl β-D-glucopyranoside (OG) (Sigma-Aldrich ref O8001) 1 M en tampon Tris 10 mM pH 7.0 pour obtenir une suspension limpide de lipides à 12 mg/ml, OG 100 mM, tampon Tris 10 mM. On poursuit l'agitation 1 hr à température ambiante sur table d'agitation. Puis on filtre stérilement sur Millex HV 0.45 µm.

On prépare dans des conditions stériles, une composition en mettant en présence du LPS et des lipides dans un rapport molaire lipides : LPS de 250 (0.160 mg/mL de LPS L8, 9.412 mg/mL de lipides et 100 mM d'OG). 40 mL d'une telle composition résulte du mélange des les préparations suivantes :
2.005 mL de tampon Tris 10 mM pH 7.0 ; 0.223 mL d'OG 100 mM en Tris 10 mM ;
31.373 mL de la suspension EDOPC : DOPE de ratio molaire 3 : 2 à 12 mg/ml en OG 100 mM Tris 10 mM ; et 6.4 mL d'une suspension stérile de LPS L8 à 1 mg/ml en OG 100 mM Tris 10 mM.

Après une heure d'agitation à température ambiante, la suspension est transférée stérilement dans 4 cassettes de dialyse stériles de 10 ml. Chaque cassette est dialysée 3 fois (24 hrs - 24 hrs - 72 hrs) contre 200 volumes de Tris 10 mM pH 7.0 soit 2 L.

On récupère les liposomes dans des conditions stériles. L'augmentation de volume après dialyse est environ de 30 %.

A cette préparation, on ajoute du merthiolate et du NaCl pour obtenir une préparation de liposomes en Tris 10 mM, NaCl 150 mM, pH 7.0, Merthiolate 0.001 % qui contient *in fine* environ 110 µg/ml de LPS et 7 mg/ml de lipides dont environ 4.5 mg/ml d'EDOPC et environ 2.5 mg/ml de DOPE (concentrations théoriques).

Les liposomes LPS sont conservés à +5°C.

### 5.2. Préparation des injectables

Les liposomes sont ajustés à la concentration requise en LPS (notamment pour les tests d'immunogénicité) en Tris 10 mM NaCl 150 mM pH 7.4. La concentration en merthiolate est maintenue à 0.001%

### 6. Préparation d'un mélange liposomes [LPSL8] + rTbpB

On mélange de la rTbpB en PBS (section 1.3.) avec des liposomes [LPS L8] (section 5.) dans un rapport poids : poids rTbpB : LPS égal à 1. Puis on ajuste en tampon Tris 10 mM, NaCl 150 mM, pH 7.4 pour obtenir une préparation dans laquelle chacun des composants (rTbpB et LPS) est concentré à 80 µg/ml. La concentration en merthiolate est maintenue à 0.001 %.

### 7. Préparation de liposomes [LPS L8 + rTbpB] (ou protéoliposomes LPS L8)

### 7.1. Préparation des protéoliposomes

Les protéoliposomes LPS L8 sont préparés par élimination de détergent par BioBeads™.

Les lipides (EDOPC : DOPE) sont tout d'abord mis sous forme de film et repris en tampon Tris 10 mM, puis dispersés en OG 100 mM et filtrés stérilement, comme décrit dans la section 5.

On prépare ensuite dans des conditions stériles, un mélange constitué de rTbpB, de LPS et de lipides dans un rapport molaire lipides : LPS de 250 et dans un rapport rTbpB : LPS poids: poids de 1 (0.160 mg/mL de rTbpB, 0.160 mg/mL de LPS L8, 9.412 mg/mL de lipides et 100 mM d'OG) : Le LPS L8 en OG 100 mM est ajouté stérilement aux lipides dispersés ; ainsi que la rTbpB telle que préparée dans la section 1.2.

Le mélange lipides/LPS/OG est ensuite traité par BioBeads™ pour éliminer l'Elugent™ et l'OG et ainsi former les liposomes.

Après une heure d'agitation à température ambiante, on ajoute des BioBeads™ activées comme décrit dans la section 1.3., à raison de 0.63 g par ml de mélange. Après une heure d'agitation à température ambiante, on récupère en condition stérile les protéoliposomes LPS qui sont ensuite conservés à +5°C.

[Note : Les protéoliposomes [rTbpB] sont préparés de la même manière mais sans ajout de LPS.]

### 7.2. Préparation des injectables

Les protéoliposomes sont ajustés en NaCl 150 mM et merthiolate 0.001 % pour obtenir les concentrations requises en rTbpB et LPS (notamment la mise en oeuvre des tests d'immunogénicité).

### 8. Quantification des lipides, du LPS et de la rTbpB en liposomes

### 8.1. Dosage des lipides par HPLC-UV

### Préparation de la gamme d'étalonnage et des échantillons à analyser

On prépare une solution-mère contenant 1 mg/mL en chloroforme de chacun des lipides EDOPC et DOPE, que l'on dilue ensuite au 10^{iéme} en ajoutant un mélange acétonitrile/eau (90/10). Cette solution-mère sert à la préparation de la gamme d'étalonnage de 2 à 50 µl/ml par dilution dans le mélange acétonitrile/eau.

Les échantillons à analyser sont dilués en acétonitrile/eau pour parvenir à une concentration finale théorique d'environ 10 µg/ml.

### Conditions analytiques

On utilise une colonne Zorbax C18 Extend, 3 ; 5 µm, 3 x 150 mm, 80A, (Agilent référence 763954-302) et pour phase mobile un mélange acétonitrile/eau/acide trifluoroacétique (TFA) dans les proportions de volume 850/150/1. La colonne est conditionnée au préalable selon le processus suivant :
- débit à 0,25 ml/min pendant 20 minutes (P = 21 bars)
- débit à 0.5 ml/min pendant 20 minutes (P = 42 bars)
- débit à 0.75 ml/min pendant 20 minutes (P = 60 bars)
- débit à 1 ml/min pendant 20 minutes (P = 80 bars)

Les mesures sont effectuées à 60°C, en injectant 10 µL de la préparation sous un débit de phase mobile de 1 ml/min. Les analytes sont détectés à DO de 200 nm.

Temps de rétention moyen de l'EDOPC : 7,7 minutes

Temps de rétention moyen de la DOPE : 11,5 minutes

### 8.2. Dosage du LPS par HPAEC-PAD

Le principe du dosage consiste à soumettre le LPS à une hydrolyse acide qui libère une molécule de KDO par molécule de LPS ; puis à séparer ce KDO libre du reste et à le quantifier par chromatographie haute performance d'échange d'ions couplé à une détection ampérométrique pulsée (HPAEC-PAD).

### Préparation de la gamme étalon et des échantillons à doser

On prépare sous un volume final de 400 µL, un blanc et gamme étalon de KDO comprise entre 42.5 et 1700 ng/mL ; ce qui correspond à une gamme étalon en LPS comprise en 613 et 24507 ng de LPS/mL. Le blanc et chacun des échantillons de la gamme contient en outre une quantité de lipides et/ou de détergent sensiblement équivalente à celle présente dans les échantillons à doser; soit *e.g.,* 0.7 mg/ml d'un mélange d'EDOPC et de DOPE dans un rapport molaire de 3 : 2 ainsi que de l'octyl glucoside 0.2 mM.

Les échantillons à doser sont préparés sous un volume final de 400 µL par dilution *e.g.* au 10^{ième} d'une préparation de liposomes à une concentration théorique en LPS de départ à 100 µg/mL.

### Hydrolyse acide

On distribue 100 µL d'une solution d'hydrolyse contenant de l'acide acétique 5 % et de l'acide glucuronique à 20 µg/mL (composé utilisé comme standart interne) préparée de manière extemporanée, dans les échantillons de la gamme étalon + blanc et dans les échantillons à doser. L'hydrolyse est poursuivie 1 hr à 100°C puis arrêtée par centrifugation à 5°C pendant 5 min.

### Extraction des lipides et du détergent

On ajoute 500 µl d'eau purifiée au produit d'hydrolyse puis 2 mL d'un mélange chloroforme /méthanol 2/1 et on mélange au vortex pendant 30 sec. On centrifuge à 4500 rpm/min pendant 10 min. Les phases aqueuses sont prélevées, séchées à 45°C pendant 2 hrs sous un débit d'azote, à 0.5 bar et reprises avec 400 µl d'eaù.

### Dosage HPAEC-PAD

Cette technique est mise en oeuvre sur une chaîne HPAEC (Dionex™) en utilisant le logiciel de pilotage Chromeleon Dionex™ pour l'acquisition et le retraitement des données. La colonne de chromatographie (Carbopac PA1 x 250 mn (Dionex™ référence 035391) est soumise à une température de 30°C. La colonne est équilibrée avec une solution d'élution (NaOH 75 mM, AcONa 90 mM) et conditionnée au préalable selon le schéma suivant :
- débit à 0,20 ml/min pendant 20 minutes (P = 270 psi)
- débit à 0.4 ml/min pendant 20 minutes (P = 540 psi)
- débit à 0.6 ml/min pendant 20 minutes (P = 800 psi)
- débit à 0.8 ml/min pendant 20 minutes (P = 1055 psi)
- débit à 1 ml/min pendant 20 minutes (P = 1300 psi)

100 µL d'un échantillon sont injectés sur la colonne sous un débit d'élution de 1 ml/min pendant 22 min.

La quantité de KDO présente dans l'échantillon est déterminée par l'intégration du pic KDO du chromatogramme. Puisqu'une mole de KDO est libérée par mole de LPS, il est possible de déterminer la concentration de LPS présente dans la préparation initiale.

### 8.3. Dosage de la rTbpB en liposomes

Les protéines ne pouvant pas être dosées en présence de lipides, le dosage s'effectue directement par SDS-PAGE sur gel Criterion 4-12 % Bis Tris 18 puits (Biorad), en tampon de migration MOPS 1xC (Biorad). Le gel est coloré au GelCode (Pierce) et les concentrations en rTbpB sont déduites par densitométrie à partir d'une gamme étalon déposées sur gel.

### 9. Etudes d'immunogénicité chez le lapin et la souris

Les différentes formulations testées ont été fabriquées comme décrit dans l'une des précédentes sections.

### 9.1. Immunisations des lapins

### Expérience n° 1

Vingt quatre lapines NZ KBL (Charles River Lab.) âgées de 7 semaines sont réparties en 5 groupes test de quatre et en 2 groupes contrôle de deux.

Les lapines dé chaque groupe reçoivent sous un volume de 0.5 ml reparti en 2 injections intramusculaires concomitantes dans les pattes, à J0 et à J21 :
- Groupe 1: du Tris 10 mM, NaCl 150mM, Tween 80 0,05 %, pH 7.4 (contrôle négatif)
- Groupe 2: des liposomes tel que préparé en tampon Tris NaCl pH 7.0
- Groupe 3: 40 µg d'endotoxoïde L8 préparé tel que décrit dans la section 3, en tampon Tris NaCl Tween 80 0,05 %, pH 7.4
- Groupe 4: 40 µg de LPS L8 formulé en liposomes, en tampon Tris NaCl, pH 7.0
- Groupe 5: 40 µg de rTbpB et 40 µg de LPS L8 formulés en liposomes, en tampon Tris NaCl / PBS pH 7.0
- Groupe 6: 40 µg de rTbpB en tampon PBS
- Groupe 7: 40 µg de rTbpB mis en mélange avec 40 µg de LPS L8 formulé en liposomes, ajustés en tampon Tris NaCl, pH 7.0.

Du sang des animaux est prélevé pour analyse à J -7, J21 (avant la deuxième injection) et à J42.

### Expérience n° 2

Vingt deux lapines NZ KBL (Charles River Lab.) âgées de 7 semaines sont réparties en 5 groupes test de quatre et en un groupe contrôle de deux.

Les lapines de chaque groupe reçoivent sous un volume de 0.5 ml reparti en 2 injections intramusculaires concomitantes dans les pattes, à J0, J 21 et J42 :
- Groupe A: 40 µg de rTbpB mis en mélange avec 40 µg de LPS L8 formulés en liposomes, en tampon Tris NaCl / PBS, pH 7.0.
- Groupe B: 10 µg de rTbpB mis en mélange avec 40 µg de LPS L8 formulés en liposomes, en tampon Tris NaCl / PBS, pH 7.0.
- Groupe C: 2.5 µg de rTbpB mis en mélange avec 40 µg de LPS L8 formulé en liposomes, en tampon Tris NaCl / PBS, pH 7.0
- Groupe D: 40 µg de rTbpB mis en mélange avec 40 µg d'endotoxoïde L8 préparé tel que décrit dans la section 3., ajustés en tampon Tris NaCl Tween 80 0.05 %, pH 7.4.
- Groupe E: 40 µg de LPS L8 formulé en liposomes, en tampon Tris NaCl, pH 7.0
- Groupe F: des liposomes en tampon Tris NaCl pH 7.0

Du sang des animaux est prélevé pour analyse à J -2, J42 (avant la deuxième injection) et à J56.

### 9.2. Immunisations des souris

Cent dix souris femelles CD1 (Charles River Lab.) âgées de 7 semaines sont réparties en 10 groupes est de dix et en 2 groupes contrôle de cinq.

Les souris de chaque groupe reçoivent par voie sous-cutanée et sous un volume de 200 µl, à J0, J 21 et J35 :
- Groupe 1: du Tris 10 mM, NaCl 150mM, Tween 80, pH 7.4 (contrôle négatif)
- Groupe 2: des liposomes tel que préparé en tampon Tris NaCl pH 7.0
- Groupe 3: 10 µg d'endotoxoïde L8 préparé tel que décrit dans la section 3, en tampon Tris NaCl Tween 80 0.05 %, pH 7.4
- Groupe 4: 10 µg de LPS L8 formulé en liposomes, en tampon Tris NaCl, pH 7.0
- Groupe 5: 10 µg de rTbpB et 10 µg de LPS L8 formulés en liposomes, en tampon Tris NaCl/PBSpH 7.0
- Groupe 6: 10 µg de rTbpB en tampon PBS
- Groupe 7: 10 µg de rTbpB mis en mélange avec 10 µg de LPS L8 formulé en liposomes, ajustés en tampon Tris NaCl, pH 7.0.
- Groupe 8: 10 µg de rTbpB adjuvantée par de l'adjuvant Incomplet de Freund et 1µg d'oligonucléotide CpG 1826 (5'-TCCATGACGTTCCTGACGTT-3' ; Coley Pharm Group, Langenfeld, Germany), en tampon PBS
- Groupe 9: 10 µg rTbpB lipidée formulée en liposomes,
- Groupe 10: 10 µg de rTbpB Cter, en tampon PBS
- Groupe 11: 10 µg de rTbpB Cter, en tampon PBS mis en mélange avec 10 µg de LPS L8 formulé en liposomes, en tampon Tris NaCl, pH 7.0
- Groupe 12: 10 µg de rTbpB Cter adjuvantée par de l'adjuvant Incomplet de Freund et 10µg d'oligonucléotide CpG 1826, en tampon PBS.

La rTbpB C-ter des groupes 10, 11 et 12 est une TbpB tronquée, dépourvue de la partie N-terminale. Elle a pour caractéristique de ne pas être lipidée.

Le sang des animaux est prélevé pour analyse à J42.

### 9.3. Dosage des anticorps and-LPS par ELISA

Ce dosage est robotisé (robot Staccato, Caliper) selon le protocole suivant :
Les puits de plaques Dynex™ de 96 puits sont imprégnés avec 1 µg de LPS L8 en tampon PBS (phosphate buffer saline) IX, pH 7.1 ° MgCl2 10mM, incubées 2 heures à 37°C puis une nuit à 4°C. On bloque les plaques en ajoutant dans les puits 150 µl de PBS contenant 0.05 % de Tween 20 et 1 % (poids/vol.) de lait écrémé en poudre (PBS-Tween-lait). Les plaques sont incubées 1 hr à 37°C.

Des dilutions en série de deux en deux des échantillons à tester sont réalisées en PBS - Tween 0.05 % - lait 1 %. Les plaques sont incubées 90 min à 37°C puis lavées 3 fois avec du PBS + Tween 20 à 0.05 %.

Un conjugué peroxydase - anti-IgG de souris ou de lapins en PBS-Tween-lait est ajouté dans les puits et les plaques sont incubées 90 min à 37°C. Les plaques sont lavées trois fois. On distribue 100 µl par puits d'une solution de TMB (3,3',5,5'-tétraméthylbenzidine, substrat de la peroxidase) prête à l'emploi. Les plaques sont incubées dans l'obscurité pendant 20 min à température ambiante. La réaction est stoppée en ajoutant 100 µl de HCl 1M par puits.

La densité optique est mesurée à 450-650 nm avec un lecteur automatique (Multiskan Ascent). En l'absence de standard, les titres anticorps sont déterminés comme étant la dilution réciproque donnant une densité optique de 1.0 sur une courbe Tendance (logiciel CodUnit). Le seuil de détection des anticorps est de 1.3 log₁₀ unité ELISA. Pour chaque titre inférieur à ce seuil, une valeur arbitraire de 1.3 logₒ₁₀ est attribuée.

### 9.4. Dosage des anticorps anti-rTbpB par ELISA

Les IgG anti-TbpB sont dosées manuellement selon le protocole suivant :
Les puits de plaques Dynex™ de 96 puits sont imprégnés avec 200 ng de TbpB complète en tampon carbonate de sodium 0.05 M, pH 9.6 (Sigma). Les plaques sont incubées une nuit à 4°C. On bloque les plaques en ajoutant dans les puits 150 µl de PBS pH 7.1 contenant 0.05 % de Tween 20 et 1 % (poids/vol.) de lait écrémé en poudre (PBS-Tween-lait). Les plaques sont incubées 1 hr à 37°C.

Des dilutions en série de deux en deux des échantillons à tester sont réalisées en PBS - Tween 0.05 % - lait 1 % ; puis ajoutées dans les puits (à partir des dilutions 1/100 ou 1/100). Les plaques sont incubées 90 min à 37°C puis lavées 3 fois avec du PBS + Tween 20 à 0.05 %.

Un conjugué peroxydase - anti-IgG de souris (Jackson ; dilué au 1/4 000 en PBS-Tween-lait) ou de lapin (Sigma ; dilué au 1/40 000 en PBS-Tween-lait) est ajouté dans les puits et les plaques sont incubées 90 min à 37°C. Les plaques sont lavées trois fois. On distribue 100 µl par puits d'une solution de TMB (3,3',5,5'-tétraméthylbenzidine, substrat dé la peroxidase) prête à l'emploi (Tebu). Les plaques sont incubées dans l'obscurité pendant 30 min à température ambiante. La réaction est arrêtée en ajoutant 100 µl de HCl 1 M par puits.

La densité optique (DO) est mesurée à 450 nm-650 nm avec un lecteur de plaques ELISA (Spectra Max). Les blancs (moyenne des contrôles négatifs) sont soustraits des données. Les titres IgG sont calculés avec le logiciel Codunit pour les valeurs de DO comprises entre 0.2 à 3, à partir de la courbe de titration (sérum standard hyperimmun de lapin déposé sur chaque plaque). Le titre IgG de ce standard, exprimé en unités ELISA, a été défini précédemment à partir de plusieurs tests indépendants, et correspond à la valeur en log10 de la moyenne arithmétique de l'inverse de la dilution générant une DO de 1 dans chacun de ces tests.

La valeur seuil de détection est de 10 unités ELISA (1 log10). Les titres finaux sont exprimés en log10.

### 9.5. Mesure de l'activité bactéricide des sérums

Les sérums sont inactivés par chauffage à 56°C pendant 30 min. On réalise 10 dilutions sérielles de raison 2 en PBS Dulbecco's gélatiné contenant des ions calcium et magnésium. Les dilutions sont réalisées en plaque 96 puits pour un volume final de 50 µL par puits.

On réalise une culture des souches de *N. meningitidis* en milieu BHI complémenté ou non pendant 2 hrs 30 ave du Desféral 50µM (agent chélateur du fer sous forme libre, qui permet l'expression de la TbpB).

Vingt cinq µL de la culture en phase exponentielle (4.10³ CFU/ml) ainsi que 25 µL de complément de bébé lapin au 1/1,5 sont ajoutés dans chaque puits. La plaque est incubée une heure à 37°C, sous agitation.

Cinquante µL du mélange de chaque puits sont ensuite déposés sur des boîtes de gélose Mueller-Hinton bioMérieux et incubées une nuit à 37°C sous 10 % de CO2. On décompte le nombre de clones.

Les témoins sont au nombre de trois :
Bactéries + complément de bébé lapin, sans sérum à tester (témoin « complément ») ;
Bactéries + complément de bébé lapin inactivé, sans sérum à tester (témoin « germes »); et
Bactéries + complément de bébé lapin inactivé + sérum à tester (témoin sérum).

On exprime le titre bactéricide comme étant l'inverse de la dilution donnant 50 % de mort bactérienne par comparaison avec le témoin « complément ».

Pour chaque groupe d'individus, le taux de séroconversion qui le caractérise est établi comme étant le rapport GMT au temps T après administration : GMT avant administration. On considère qu'il y a une activité bactéricide lorsque le taux de séroconversion est supérieur ou égal à 4.

### 9.6. Résultats et Discussion

### ELISAs

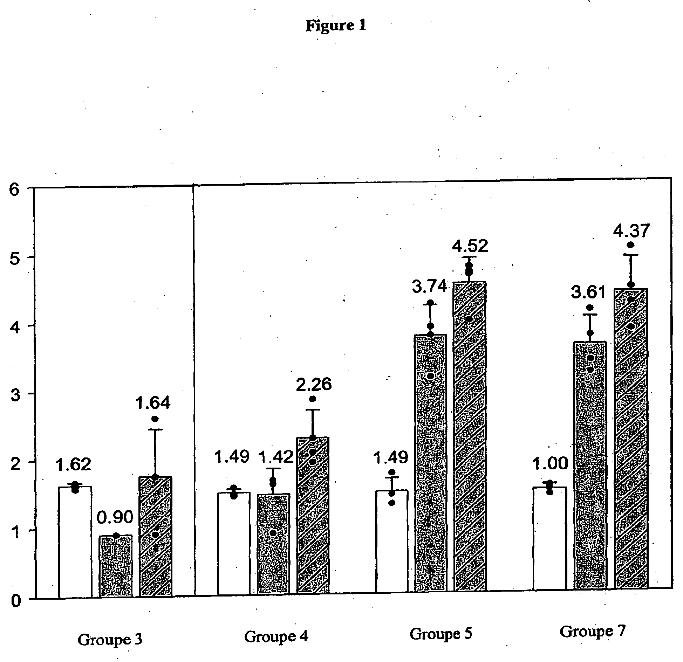

La figure 1 présente les titres ELISAs exprimés en log₁₀ des IgG anti-LPS L8 des sérums de lapins des groupes 3, 4, 5 et 7, issus de l'expérience n°1. En blanc, les titres des sérums avant immunisation ; en grisé, ceux des sérums prélevés à J21 après la première immunisation ; et en grisé /hachuré, ceux des sérums prélevés à J42.

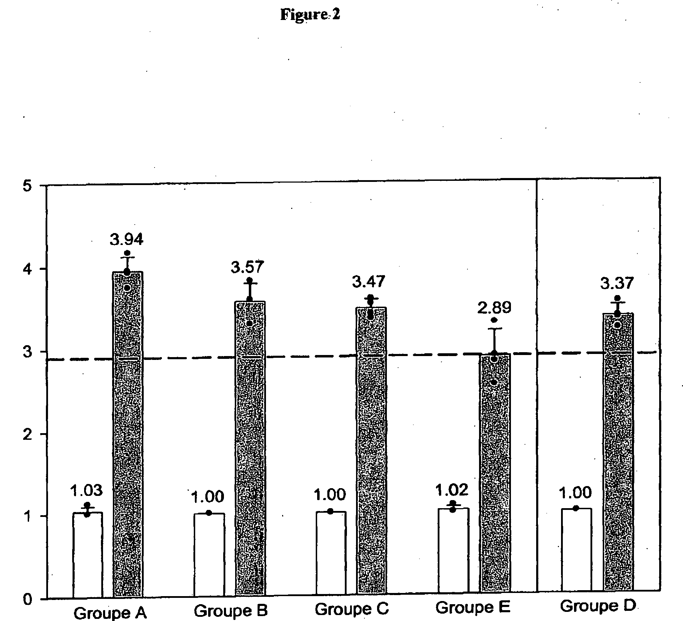

La figure 2 présente les titres ELISAs exprimés en log₁₀ des IgG anti-LPS L8 des sérums de lapins des groupes A à E, issus de l'expérience n°2. En blanc, les titres des sérums avant immunisation ; et en grisé, ceux des sérums prélevés à J56 après la première immunisation.

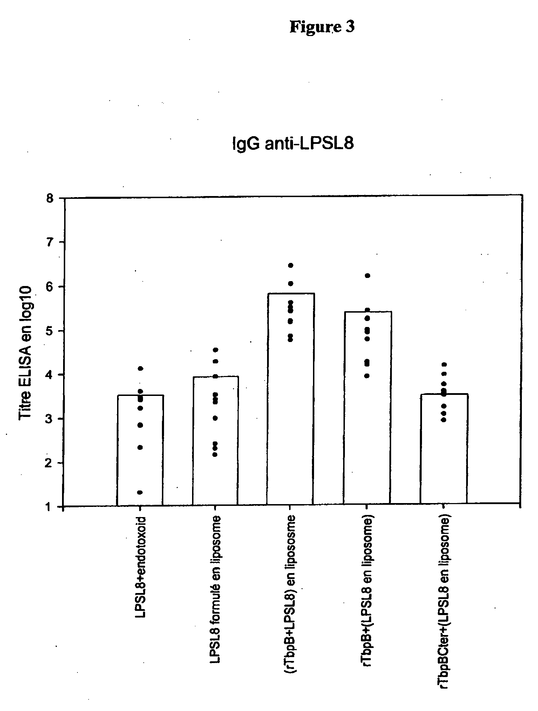

La figure 3 présente les titres ELISAs exprimés en log₁₀ des IgG anti-LPS L8 des sérums de souris.

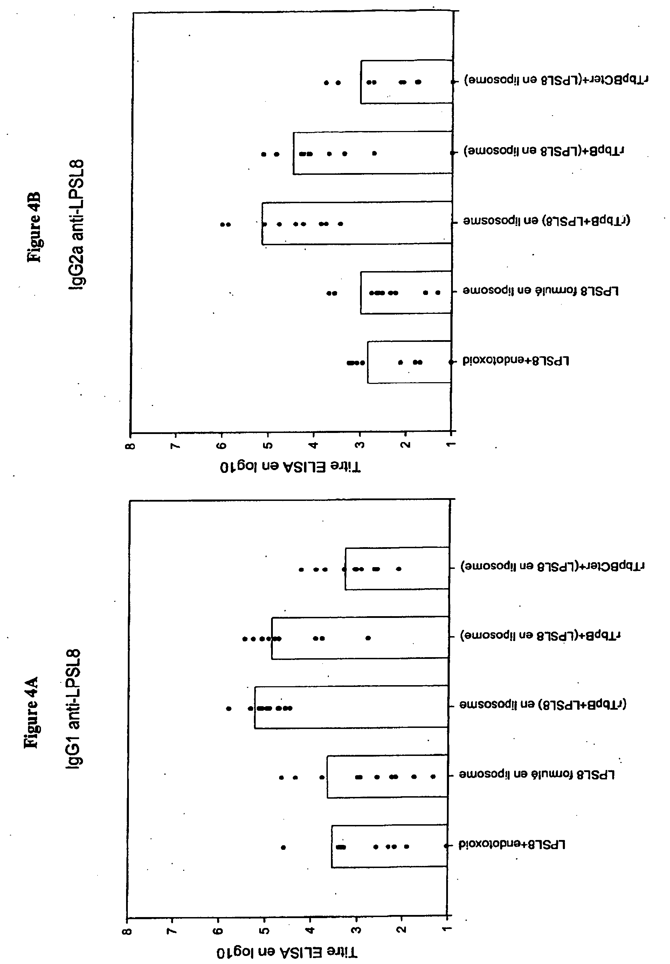

Les figures 4A et 4B présentent de manière respective, les titres ELISAs exprimés en log₁₀ des IgG1 et des IgG2a anti-LPS L8 des sérums de souris.

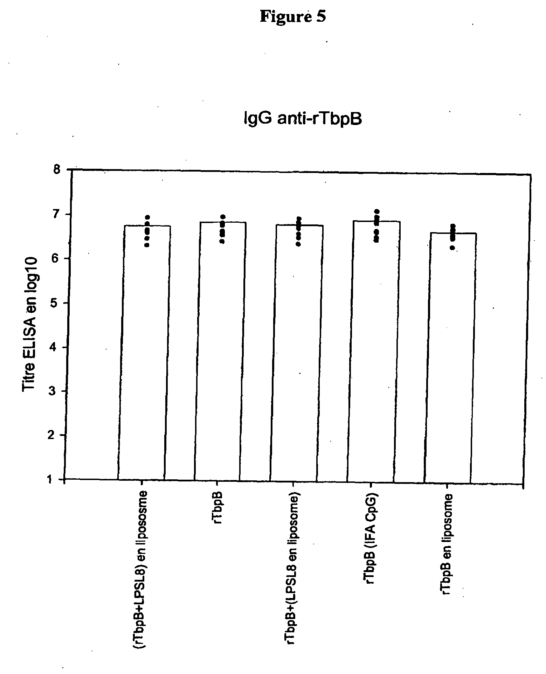

La figure 5 présente les titres ELISAs exprimés en log₁₀ des IgG anti-TbpB des sérums de souris.

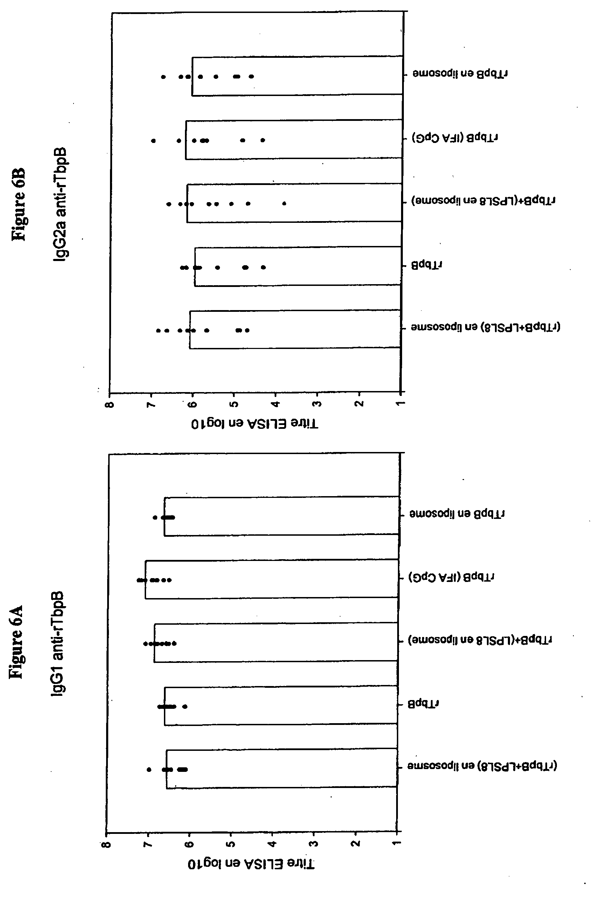

Les figures 6A et 6B présentent de manière respective, les titres ELISAs exprimés en log₁₀ des IgG1 et des IgG2a anti-TbpB des sérums de souris.

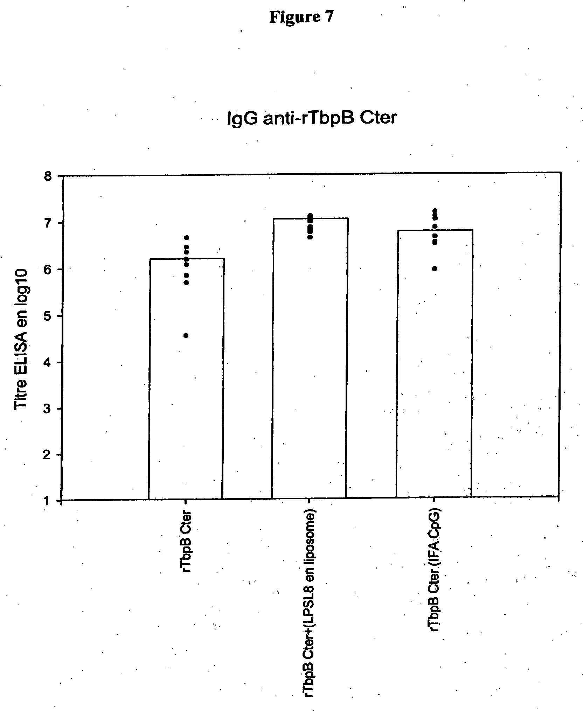

La figure 7 présente les titres ELISAs exprimés en log₁₀ des IgG anti-TbpB C-ter des sérums de souris.

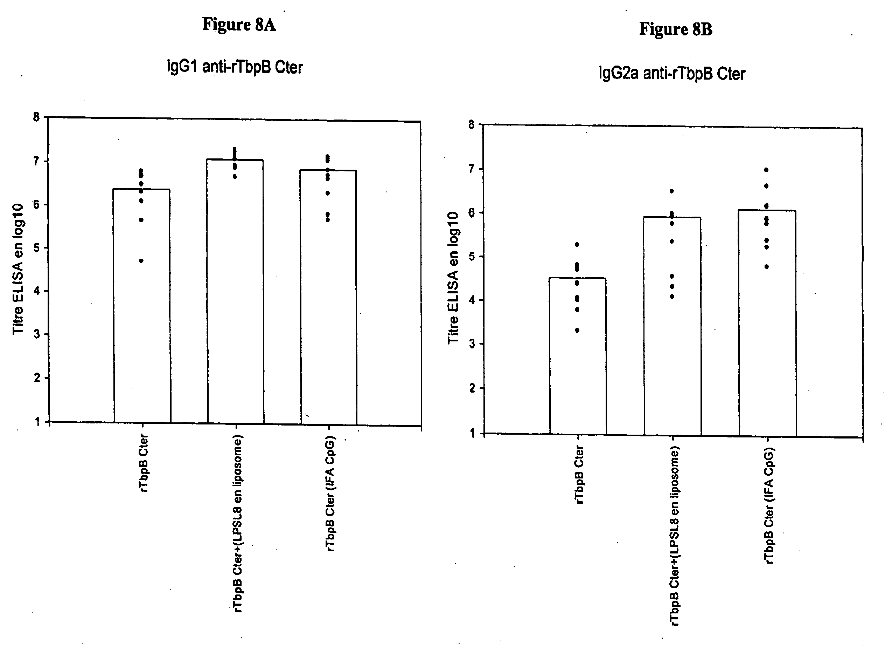

Les figures 8A et 8B présentent de manière respective, les titres ELISAs exprimés en log₁₀ des IgG1 et des IgG2a anti-TbpB C-ter des sérums de souris.

Les titres ELISAs montrent que la rTbpB lipidée et la partie C-terminale de TbpB (TbpB Cter, qui n'est pas lipidée) sont fortement antigéniques chez le lapin aussi bien que chez la souris. Par contre, seule la rTbpB lipidée possède un fort effet adjuvant sur le LPS L8. Cet effet adjuvant est observé (i) lorsque la rTbpB lipidée est simplement mis en mélange avec des liposomes [LPS L8] ou (ii) lorsque la rTbpB lipidée est mélangée à du LPS L8 puis l'ensemble formulé en liposomes ; toutefois, ce dernier mode de réalisation semble donner des résultats légèrement supérieurs. L'effet adjuvant de la rTbpB lipidée est aussi observé avec l'endotoxoïde L8. En effet, dans l'expérience n°1 en l'absence de rTbpB lipidée, l'endotoxoide L8 produit des effets similaires à ceux des liposomes [LPS L8] tandis que dans l'expérience n°2, l'endotoxoïde L8 mélangé à de la rTbpB lipidée produit des effets supérieurs à ceux des liposomes [LPS L8] et comparables à ceux des liposomes [LPS L8] + rTbpB lipidée.

Le LPS ne présente pas d'effet adjuvant sur la rTbpB lipidée probablement du à un effet « plateau » spécifique à cet antigène.

### Test de bactéricidie (sérums de lapins)

Dans les expériences n° et 2, les sérums de lapins ont été testés vis-à-vis des souches RH873 (souche d'immunotype L8, isotype TbpB II) cultivée en présence et absence de Desféral et M982 (souche d'immunotype L3, isotype TbpB II) cultivée en présence de Desféral. Les données de bactéricidie sont présentées dans les tableaux ci-dessous :

**Tableau n° 1 : Expérience n°1- Titre bactéricide des sérums de lapins prélevés à J42**

| Groupe d'immunisation | NZ KBL rabbit # | Prélèvement | *N. meningitidis* B RH873 | | | *N. meningitidis* B M982 |
|---|---|---|---|---|---|---|
| | | | BHI + 50 µM Desféral 2H30 | BHI + 50 µM Desféral 4H00 | BHI 4H00 | BHI + 50 µM Desféral 2H30 |
| | 5 | J-7 | <4 | | | [32] |
| | | J42 | 8 | | | [32] |
| Groupe 3 Endotoxoïde LPS L8 | 6 | J-7 | <4 | | | [32] |
| | | J42 | <4 | | | [16] |
| | 7 | J-7 | <4 | | | [64] |
| 40 µg | | J42 | [4] | | | [64] |
| | 8 | J-7 | [4] | | | [32] |
| | | J42 | [4] | | | [16] |
| | 9 | J-7 | <4 | | | [8] |
| Groupe 4 [LPS L8] liposome 40 µg | | J42 | <4 | | | [16] |
| | 10 | J-7 | <4 | | | [64] |
| | | J42 | 16 | | | [64] |
| Groupe 4 [LPS L8] liposome 40 µg | 11 | J-7 | <4 | | | [32] |
| | | J42 | [8] | | | [64] |
| | 12 | J-7 | 4 | | | 64 |
| | | J42 | [4] | | | [32] |
| | 13 | J-7 | <4 | [4] | <4 | [32] |
| | | J42 | 64 | 256 | 64 | 256 |
| Groupe 5 [rTbpB M982 + LPS L8] liposome | 14 | J-7 | <4 | [4] | <4 | [16] - [16] |
| | | J42 | 128 | ≥2048 | 512 | 1024 - 2048 |
| | 15 | J-7 | <4 | [4] | [4] | [32] - [8] |
| 40 µg + 40 µg | | J42 | 256 | 512 | 64 | ≥2048-2048 |
| | 16 | J-7 | <4 | [4] | <4 | [64] |
| | | J42 | 256 | 512 | 128 | 512 |
| Groupe 6 rTbpB M982 40 µg | 17 | J-7 | <4 | [4] | <4 | [64] - [16] |
| | | J42 | [4] | 8 | [4] | 1024 - 2048 |
| | 18 | J-7 | <4 | [8] | [4] | [64] - [32] |
| | | J42 | 8 | 32 | <4 | 1024-1024 |
| Groupe 6 rTbpB M982 40 µg | 19 | J-7 | <4 | [4] | [4] | [32] - [64] |
| | | J42 | 8 | 8 | [4] | ≥2048 - 1024 |
| | 20 | J-7 | <4 | <4 | <4 | [128] - [16] |
| | | J42 | 32 | 128 | [4] | ≥2048 - 1024 |
| | 21 | J-7 | <4 | [4] | <4 | [512] - [8] |
| | | J42 | 64 | 256 | 32 | ≥2048 - 1024 |
| Groupe 7 rTbpB M982 + [LPS L8] liposome | 22 | J-7 | <4 | [4] | <4 | [64] - [8] |
| | | J42 | 128 | 1024 | 32 | ≥2048 - 1024 |
| | 23 | J-7 | <4 | [8] | <4 | [128] - [8] |
| 40 µg + 40 µg | | J42 | 128 | 512 | 32 | >2048 - 1024 |
| | 24 | J-7 | <4 | [4] | <4 | [256] - 8 |
| | | J42 | 512 | 1024 | 256 | >2048 - 1024 |
| Groupe 1 Contrôle tampon Tris NaCl Tween pH7,4 | 1 | J-7 | <4 | | | [32] |
| | | J42 | <4 | | | 128 |
| | 2 | J-7 | <4 | | | [32] |
| | | J42 | <4 | | | [32] |
| Groupe 2 Contrôle liposome Tris NaCl pH 7,4 | 3 | J-7 | <4 | <4 | <4 | [16] |
| | | J42 | <4 | [4] | <4 | [32] |
| | 4 | J-7 | <4 | <4 | <4 | [16] |
| | | J42 | <4 | [4] | <4 | [32] |

**Tableau 2 : Expérience n°1 - Synthèse des résultats**

| **Groupe d'immunisation** | **Données de bactéricidie** | **RH873 BHI + 50 film Desféral 2H30** | **RH873 BHI 4H00** | **M982 BHI + 50 µM Desféral 2H30** |
|---|---|---|---|---|
| Groupe 3 Endotoxoïde L8 | GMT et % de répondeurs dont le rapport des titres bactéricides à J 42 / J-7 est ≥ 4 | 4 (25%) | ND | 27 (0%) |
| | Séroconversion à J42 (comparée à J-7) | x 1,7 | ND | x 0,7 |
| Groupe 4 [LPS L8] liposome | GMT et % de répondeurs dont le rapport des titres bactéricides à J 42 / J-7 est ≥ 4 | 5,6 (50%) | ND | 38 (0%) |
| | Séroconversion à J42 (comparée à J-7) | x 2,3 | ND | x 1,2 |
| Groupe 5 [rTbpB M982 + LPS L8] liposome | GMT et % de répondeurs dont le rapport des titres bactéricides à J 42 / J-7 est ≥ 4 | 152 (100%) | 128 (100%) | 724 (100%) |
| | Séroconversion à J42 (comparée à J-7) | x 76 | x 56 | X 27 |
| Groupe 6 rTbpB M982 | GMT et % de répondeurs dont le rapport des titres bactéricides à J 42 / J-7 est ≥ 4 | 9,5 (75%) | 3,3 (0%) | 1448 (100%) |
| | Séroconversion à J42 (comparée à J-7) | x 4,7 | x 1,2 | x23 |
| Groupe 7 rTbpB M982 + [LPS L8] liposome | GMT et % de répondeurs dont le rapport des titres bactéricides à J 42 / J-7 est ≥ 4 | 152 (100%) | 54 (100%) | 2896 (100%) |
| | Séroconversion à J42 (comparée à J-7) | x 76 | x 27 | x 16 |
| Groupe 1 Buffer Tris NaCl Tween | GMT et % de répondeurs dont le rapport des titres bactéricides à J 42 / J-7 est ≥ 4 | 2 (0%) | ND | 64 (0%) |
| | Séroconversion à J42 (comparée à J-7) | x1 | ND | x2 |
| Groupe 2 Buffer Liposome | GMT et % de répondeurs dont le rapport des titres bactéricides à J 42 / J-7 est ≥ 4 | 2 (0%) | 2 (0 %) | 32 (0%) |
| | Séroconversion à J42 (comparée à J-7) | x1 | x2 | x2 |

On observe une activité bactéricide des anticorps vis-à-vis de la souche M982 dans tous les groupes ayant reçus de la rTbpB M982. Ceci confirme que la rTbpB. possède une activité bactéricide intrinsèque contre la souche homologue.

On observe aussi une importante activité bactéricide des anticorps vis-à-vis de la souche RH873. dans les 2 groupes ayant reçu des protéosomes ou de la rTbpB en mélange avec des liposomes LPS L8. Le fait que le titre bactéricide du groupe TbpB M982 seul soit faible vis-à-vis de RH873 et que les titres bactéricides observés vis-à-vis de la souche RH873 cultivée en absence de Desféral (pas d'expression de TbpB) soient peu différents de ceux obtenus en milieu chélaté (expression de TbpB), révèle que la bactéricidie observée vis-à-vis de cette souche est due en grande majorité à des anticorps anti LPS L8 et que la présence de rTbpB M982 (intégrée ou non en liposome) permet d'adjuver la réponse anti LPS L8.

**Tableau 3 : Expérience n°2 - Synthèse des résultats**

| **Groupe d'immunisation** | **Données de bactéricidie** | **RH873 BHI + 50 µM Desféral 2H30** | **RH873 BHI 4h00** | **M982 BHI + 50 µM Desféral 2H30** |
|---|---|---|---|---|
| [LPS L8] liposomes + rTbpB (40 µg) | GMT et % de répondeurs dont le rapport des titres bactéricides à J 56 / J-2 est ≥ 4 | 152 (100%) | 54 (100%) | 2896 (100%) |
| | Séroconversion à J56 (comparée à J-2) | x63 | x 16 | x256 |
| [LPS L8] liposomes + rTbpB0 µg) | GMT et % de répondeurs dont le rapport des titres bactéricides à J 56 / J-2 est ≥ 4 | 181 (100%) | 108 (100%) | 3444 (100%) |
| | Séroconversion à J56 (comparée à J-2) | x65 | x27 | x 305 |
| [LPS L8] liposomes + rTbpB (2,5 µg) | GMT et % de répondeurs dont le rapport des titres bactéricides à J 56 / J-2 est ≥ 4 | 181 (100%) | 128(100%) | 2896 (100%) |
| | Séroconversion à J56 (comparée à J-2) | x 75 | x 27 | x 305 |
| Endotoxoid LPS L8 + rTbpB (40 µg) | GMT et % de répondeurs dont le rapport des titres bactéricides à J 56 / J-2 est ≥ 4 | 181 (100%) | 152 (100%) | 2896 (100%) |
| | Séroconversion à J56 (comparée à J-2) | x 5 | x 45 | x 256 |
| [LPS L8] liposomes | GMT et % de répondeurs dont le rapport des titres bactéricides à J 56 / J-2 est ≥ 4 | 22,7 (100%) | 38 (100%) | 23 (0%) |
| | Séroconversion à J56 (comparée à J-2) | x 8,1 | x11 | x 1,4 |
| Liposomes vides | GMT et % de répondeurs dont le rapport des titres bactéricides à J 56 / J-2 est ≥ 4 | 4 (0%) | 4(0%) | 16 (0%) |
| | Séroconversion à J56 (comparée à J-2) | x2 | x2 | x 1,4 |

| | | | | |
|---|---|---|---|---|
| Les résultats obtenus dans l'expérience n°2 confirment ceux déjà obtenus : à savoir la mise en évidence de l'effet adjuvant de rTbpB (données non fournies). Chez le lapin, cet effet est déjà maximal à la dose de 2.5 µg. L'effet adjuvant s'exerce aussi sur l'endotoxoïde. | | | | |

## Revendications

1. Un procédé pour adjuver le lipopolysaccharide (LPS) d'une bactérie à Gram-négatif, selon lequel :
(i) on met en mélange du LPS ou des liposomes LPS (LPS formulé en liposomes) avec la sous-unité B lipidée du récepteur de la transferrine humaine (TbpB lipidée) de *Neisseria meningitidis* ou un fragment lipidé de ladite sous-unité B ; ou
(ii) on formule ensemble en liposomes du LPS et la TbpB lipidée de *N. meningitidis* ou un fragment lipidé de ladite TbpB ; ou
(iii) on conjugue du LPS avec la TbpB lipidée de *N. meningiridis* ou un fragment lipidé de ladite TbpB ;
et dans lequel chacunes des alternatives (i), (ii) et (iii) permet d'obtenir une préparation qui ne contient pas de vésicules de membrane externe (OMVs) et qui est capable d'induire après administration à un mammifère, une réponse immune anti-LPS qui est améliorée par comparaison à la réponse immune anti-LPS observée après administration de la préparation correspondante dans laquelle la TbpB lipidée de *N. meningitidis* ou un fragment lipidé de cette dernière est omis.

2. Un procédé selon la revendication 1, dans lequel le LPS est le LOS de *N. meningitidis.*

3. Un procédé selon la revendication 1 ou 2, pour adjuver du LPS selon lequel :
(i) on met en mélange du LPS détoxifié avec la TbpB lipidée ou un fragment lipidé de ladite TbpB ; ou
(ii) on conjugue du LPS détoxifié avec la TbpB lipidée ou un fragment lipidé de ladite TbpB.

4. Un procédé selon la revendication 1 ou 2, pour adjuver du LPS selon lequel :
(i) on met en mélange des liposomes LPS (LPS formulé en liposomes) avec la TbpB lipidée ou un fragment lipidé de ladite TbpB ; ou
(ii) on formule en liposomes du LPS et la TbpB lipidée ou un fragment lipidé de ladite TbpB.

5. Un procédé selon la revendication 4, dans lequel la matrice des liposomes est constituée par un mélange de lipide cationique et de lipide neutre.

6. Un vaccin qui ne contient pas d'OMVs et qui comprend (i) un antigène vaccinal qui est le LPS d'une bactérie à Gram-négatif, optionnellement formulé en liposomes ou conjugué à un polypeptide porteur ou sous forme d'endotoxoïde, et (ii) un adjuvant de la réponse immune anti-LPS qui est la TbpB lipidée de *N. meningitidis* ou un fragment lipidé de ladite TbpB.

7. Un vaccin selon la revendication 6, dans lequel le LPS est le LOS de *N. meningitidis.*

8. Un vaccin selon la revendication 7, dans lequel le LPS est le LOS d'une souche de *N. meningitidis* d'immunotype L8.

9. Un vaccin selon la revendication 7, dans lequel le LPS est le LOS de la souche A1 de *N. meningitidis.*

10. Un vaccin selon l'une des revendications 6 à 9, dans lequel le LPS est formulé en liposomes.

11. Un vaccin selon l'une des revendications 6 à 10, dans lequel le LPS et la TbpB lipidée de *N. meningitidis* ou un fragment lipidé de ladite TbpB sont formulés ensemble en liposomes.

12. Un vaccin selon l'une des revendications 6 à 9, dans lequel le LPS est sous forme d'endotoxoïde.

13. Un vaccin selon l'une des revendications 6 à 9, dans lequel le LPS est conjugué à un polypeptide porteur. -

14. Un vaccin selon l'une des revendications 6 à 9, dans lequel le LPS est conjugué à la TbpB lipidée de *N. meningitidis* ou un fragment lipidé de ladite TbpB.

15. Un vaccin selon l'une des revendications 6 à 14, qui ne contient pas de composé ayant une fonction d'adjuvant autre que le LPS et la TbpB lipidée de *N. meningitidis* ou un fragment lipidé de cette dernière.

## Patentansprüche

1. Verfahren zur Adjuvierung des Lipopolysaccharids (LPS) eines gramnegativen Bakteriums, bei dem man:
(i) LPS oder LPS-Liposomen (zu Liposomen formuliertes LPS) mit der lipidierten Untereinheit B des menschlichen Transferinrezeptors (mit lipidierter TbpB) von *Neisseria meningitidis* oder einem lipidierten Fragment dieser Untereinheit B mischt; oder
(ii) LPS und lipidierte TbpB aus *N. meningitidis* oder ein lipidiertes Fragment der TbpB gemeinsam zu Liposomen formuliert; oder
(iii) LPS mit lipidierter TbpB aus *N. meningitidis* oder einem lipidiertem Fragment dieser TbpB konjugiert;
und wobei es jede der Alternativen (i), (ii) und (iii) ermöglicht, ein Präparat zu erhalten, das keine Vesikel der äußeren Membran (OMV) enthält und das fähig ist, nach Verabreichen an einen Säuger eine Anti-LPS-Immunreaktion hervorzurufen, die im Vergleich zu der nach Verabreichung des entsprechenden Präparats, in dem die lipidierte TbpB aus *N. meningitidis* oder ein lipidiertes Fragment davon nicht vorliegt, beobachteten Anti-LPS-Immunreaktion verbessert ist.

2. Verfahren nach Anspruch 1, wobei es sich bei dem LPS um das LOS von *N. meningitidis* handelt.

3. Verfahren nach Anspruch 1 oder 2 zum Adjuvieren von LPS, bei dem man:
(i) detoxifiziertes LPS mit der lipidierten TbpB oder einem lipidierten Fragment der TbpB mischt; oder
(ii) detoxifiziertes LPS mit der lipidierten TbpB oder einem lipidierten Fragment der TbpB konjugiert.

4. Verfahren nach Anspruch 1 oder 2 zum Adjuvieren von LPS, bei dem man:
(i) LPS-Liposomen (zu Liposomen formuliertes LPS) mit der lipidierten TbpB oder einem lipidierten Fragment der TbpB mischt; oder
(ii) LPS und die lipidierte TbpB oder ein lipidiertes Fragment der TbpB zu Liposomen formuliert.

5. Verfahren nach Anspruch 4, wobei die Matrix der Liposomen aus einer Mischung aus kationischem Lipid und neutralem Lipid besteht.

6. Impfstoff, der keine OMV enthält und der (i) ein Impfstoffantigen, bei dem es sich um das LPS eines gramnegativen Bakteriums handelt, das gegebenenfalls zu Liposomen formuliert ist oder an einem Trägerpolypeptid konjugiert ist oder in Form des Endotoxoids vorliegt, und (ii) ein Adjuvans der Anti-LPS-Immunreaktion, bei dem es sich um die lipidierte TbpB von *N. meningitidis* oder ein lipidiertes Fragment der TbpB handelt, umfasst.

7. Impfstoff nach Anspruch 6, wobei es sich bei dem LPS um das LOS von *N. meningitidis* handelt.

8. Impfstoff nach Anspruch 7, wobei es sich bei dem LPS um das LOS eines *N*. *meningitidis*-Stamms des Immuntyps L8 handelt.

9. Impfstoff nach Anspruch 7, wobei es sich bei dem LPS um das LOS des *N. meningitidis*-Stamms A1 handelt.

10. Impfstoff nach einem der Ansprüche 6 bis 9, wobei das LPS zu Liposomen formuliert ist.

11. Impfstoff nach einem der Ansprüche 6 bis 10, wobei das LPS und die lipidierte TbpB von *N*. *meningitidis* oder ein lipidiertes Fragment der TbpB gemeinsam zu Liposomen formuliert sind.

12. Impfstoff nach einem der Ansprüche 6 bis 9, wobei das LPS in Form eines Endotoxoids vorliegt.

13. Impfstoff nach einem der Ansprüche 6 bis 9, wobei das LPS an einem Trägerpolypeptid konjugiert ist.

14. Impfstoff nach einem der Ansprüche 6 bis 9, wobei das LPS an der lipidierten TbpB von *N*. *meningitidis* oder einem lipidierten Fragment der TbpB konjugiert ist.

15. Impfstoff nach einem der Ansprüche 6 bis 14, der außer dem LPS und der lipidierten TbpB von *N*. *meningitidis* oder einem lipidierten Fragment der letztgenannten keine Verbindung mit Adjuvansfunktion enthält.

## Claims

1. Method for adjuvanting lipopolysaccharide (LPS) from a Gram-negative bacterium, according to which:
(i) LPS or LPS liposomes (LPS formulated in liposomes) is (are) mixed with the lipidated human transferring receptor subunit B (lipidated TbpB) from *Neisseria meningitidis* or a lipidated fragment of said subunit B; or
(ii) LPS and the lipidated TbpB from *N*. *meningitidis* or a lipidated fragment of said TbpB are formulated together in liposomes; or
(iii) LPS is conjugated with the lipidated TbpB from *N. meningitidis* or a lipidated fragment of said TbpB;
and in which each of the alternatives (i), (ii) and (iii) makes it possible to obtain a preparation which does not contain any outer membrane vesicles (OMVs) and which is capable of inducing, after administration to a mammal, an anti-LPS immune response which is improved by comparison with the anti-LPS immune response observed after administration of the corresponding preparation in which the lipidated TbpB from *N*. *meningitidis* or a lipidated fragment thereof is omitted.

2. Method according to Claim 1, in which the LPS is LOS from *N*. *meningitidis.*

3. Method according to Claim 1 or 2, for adjuvanting LPS, according to which:
(i) detoxified LPS is mixed with the lipidated TbpB or a lipidated fragment of said TbpB; or
(ii) detoxified LPS is conjugated with the lipidated TbpB or a lipidated fragment of said TbpB.

4. Method according to Claim 1 or 2, for adjuvanting LPS, according to which:
(i) LPS liposomes (LPS formulated in liposomes) are mixed with the lipidated TbpB or a lipidated fragment of said TbpB; or
(ii) LPS and the lipidated TbpB or a lipidated fragment of said TbpB are formulated in liposomes.

5. Method according to Claim 4, in which the liposome matrix is made up of a mixture of cationic lipid and neutral lipid.

6. Vaccine which does not contain OMVs and which comprises (i) a vaccine antigen which is the LPS from a Gram-negative bacterium, optionally formulated in liposomes or conjugated to a carrier polypeptide or in endotoxoid form, and (ii) an adjuvant of the anti-LPS immune response, which is a lipidated TbpB from *N. meningitidis* or a lipidated fragment of said TbpB.

7. Vaccine according to Claim 6, in which the LPS is LOS from *N. meningitidis.*

8. Vaccine according to Claim 7, in which the LPS is LOS from a strain of *N. meningitidis* of immunotype L8.

9. Vaccine according to Claim 7, in which the LPS is LOS from the *N. meningitidis* strain A1.

10. Vaccine according to one of Claims 6 to 9, in which the LPS is formulated in liposomes.

11. Vaccine according to one of Claims 6 to 10, in which the LPS and the lipidated TbpB from *N*. *meningitidis* or a lipidated fragment of said TbpB are formulated together in liposomes.

12. Vaccine according to one of Claims 6 to 9, in which the LPS is in endotoxoid form.

13. Vaccine according to one of Claims 6 to 9, in which the LPS is conjugated to a carrier polypeptide.

14. Vaccine according to one of Claims 6 to 9, in which the LPS is conjugated to the lipidated TbpB from *N. meningitidis* or a lipidated fragment of said TbpB.

15. Vaccine according to one of Claims 6 to 14, which does not contain any compound that has an adjuvant function other than the LPS and the lipidated TbpB from *N. meningitidis* or a lipidated fragment thereof.
